(19)

Europäisches
Patentamt

European
Patent Office

Office européen
des brevets

(11) **EP 4 711 453 A1**

(12) **EUROPEAN PATENT APPLICATION**
published in accordance with Art. 153(4) EPC

(43) Date of publication:
**18.03.2026 Bulletin 2026/12**

(21) Application number: 24802896.1

(22) Date of filing: **30.04.2024**

(51) International Patent Classification (IPC):
***C12N 9/88*** (2006.01)     ***C12N 9/96*** (2006.01)
***A61K 38/43*** (2006.01)

(52) Cooperative Patent Classification (CPC):
**A61K 38/43; A61K 38/51; A61K 47/60; A61P 3/00;**
**A61P 35/00; A61P 35/02; C12N 9/88; C12N 9/96**

(86) International application number:
**PCT/CN2024/091015**

(87) International publication number:
**WO 2024/230614 (14.11.2024 Gazette 2024/46)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **09.05.2023  CN 202310520154**

(71) Applicant: **Peg-Bio Biopharm Co., Ltd.**
**(Chongqing)**
**Chongqing 400714 (CN)**

(72) Inventors:
• **FAN, Kai**
  **Chongqing 400714 (CN)**
• **FU, Zhicheng**
  **Chongqing 400714 (CN)**
• **TANG, Guanghui**
  **Chongqing 400714 (CN)**

• **WU, Kuncan**
  **Chongqing 400714 (CN)**
• **ZENG, Xin**
  **Chongqing 400714 (CN)**
• **LUAN, Qiuyang**
  **Chongqing 400714 (CN)**
• **LIU, Riyong**
  **Chongqing 400714 (CN)**
• **CHEN, Qing**
  **Chongqing 400714 (CN)**

(74) Representative: **Rigamonti, Dorotea et al**
**THINX S.r.l.**
**Piazzale Luigi Cadorna, 10**
**20123 Milano (IT)**

Remarks:
The complete document including Reference
Table(s) and the Sequence Listing(s) can be
downloaded from the EPO website

(54) **PHENYLALANINE LYASE MUTANT, AND CONJUGATE THEREOF OR PHARMACEUTICALLY**
**ACCEPTABLE SALT THEREOF**

(57)    Provided is a phenylalanine lyase mutant. Compared with the amino acid sequence of phenylalanine lyase, the phenylalanine lyase mutant has a mutation at at least one of the following sites: site 72, site 240, site 467 and site 544.

**EP 4 711 453 A1**

**Description**

## TECHNICAL FIELD

The present disclosure belongs to the field of biopharmaceutical technology. Specifically, the present disclosure relates to a phenylalanine lyase mutant and a conjugate thereof or a pharmaceutically acceptable salt thereof. More particularly, the present disclosure relates to a phenylalanine lyase mutant, a nucleic acid molecule, an expression vector, a recombinant cell, a conjugate of the phenylalanine lyase mutant or a pharmaceutically acceptable salt thereof, a polyethylene glycol-modified phenylalanine lyase mutant, a pharmaceutical composition and uses thereof, and a method for reducing immunogenicity while maintaining enzymatic activity of phenylalanine lyase.

## BACKGROUND

Phenylketonuria (PKU), also known as phenylalanine hydroxylase deficiency, is a metabolic disorder caused by a deficiency or reduced activity of phenylalanine hydroxylase in the liver and is a recessive genetic disease. Phenylalanine is an essential amino acid that participates in various physiological activities. However, it cannot be synthesized by the human body and must be ingested from food. Under normal conditions, the ingested phenylalanine, in addition to being utilized for the synthesis of various proteins, is converted into tyrosine under the catalysis of phenylalanine hydroxylase, and subsequently transformed by other enzymes into compounds such as L-DOPA, dopamine, epinephrine, norepinephrine, or melanin. Patients with phenylketonuria are unable to effectively degrade phenylalanine due to a deficiency or reduced activity of phenylalanine hydroxylase, resulting in accumulation of phenylalanine in plasma. When the plasma phenylalanine concentration exceeds the renal threshold, it is excreted in urine, leading to phenylalaninuria. In addition, the accumulated phenylalanine is metabolized via alternative pathways to produce toxic substances such as phenylpyruvate and phenyllactate, which are harmful to the brain and other components of the nervous systems.

This disease is a recessive genetic disease. After birth, affected infants lack phenylalanine hydroxylase (PAH) activity and cannot effectively degrade phenylalanine derived from milk or diet, leading to the accumulation of neurotoxic substances such as phenylpyruvate, thereby impairing the development of the brain and other components of the nervous system. As the child ages, severe intellectual disability develops, along with other abnormal behaviors such as epilepsy. Impaired PAH activity in the liver disrupts phenylalanine metabolism, leading to phenylketonuria (PKU) and hyperphenylalaninemia (HPA), which manifest as impaired neurophysiological function and delayed cognitive development. If a pregnant woman suffers from phenylketonuria, neurological damage in the fetus may begin to occur in utero.

Currently, there are three main treatments for phenylketonuria: dietary control therapy, BH4 (tetrahydrobiopterin) replacement therapy, and pharmacological therapy.

Dietary control therapy reduces the accumulation of phenylalanine and phenylpyruvate in plasma by lowering dietary phenylalanine intake, and is supplemented with 5-hydroxytryptamine and L-dopamine treatment. Although this approach can control symptoms, long-term patient compliance and quality of life are difficult to maintain. Moreover, it cannot eliminate the neurological impairments caused by elevated phenylalanine levels. Dietary control during pregnancy may also lead to congenital defects in the fetus, and nutritional deficiencies during dietary treatment in children can likewise be detrimental to brain development.

BH4 replacement therapy reduces phenylalanine concentrations in the body by administering a physiological dose of BH4, but it is not effective in all patients. Currently, only two drugs developed by BioMarin Pharmaceutical Inc. and Suntory Co., Ltd. (Japan) have been approved for the treatment of phenylketonuria. However, both drugs are indicated only for atypical hyperphenylalaninemia (i.e., hyperphenylalaninemia caused by deficiency of dihydropteridine reductase or synthetase), and are not effective for phenylketonuria caused by phenylalanine hydroxylase deficiency.

Therefore, there is an urgent need to develop a pharmaceutical agent capable of effectively treating hyperphenylalaninemia-related diseases such as phenylketonuria.

## SUMMARY

The present disclosure is directed, at least in part, to addressing at least one of the technical problems associated with the prior art. To this end, the present disclosure provides a phenylalanine lyase mutant conjugate having the advantages of low immunogenicity and a long in vivo half-life, which can be used for the prevention and treatment of hyperphenylalaninemia-related diseases such as phenylketonuria.

The present disclosure has been accomplished based on the inventors' following findings:

Phenylalanine lyase is a non-mammalian enzyme widely found in plants (e.g., *Rhodotorula toruloides, Rhodotorula rubra, Solanum tuberosum* (potato), *Triticum aestivum* (wheat), lichens, *Streptomyces* species, and *Anabaena variabilis*). Its active form is a homotetramer that converts phenylalanine into trans-cinnamic acid and ammonia, which is further metabolized to hippuric acid and then excreted in the urine from the body. As early as the late 1990s, researchers had administered phenylalanine lyase derived from *Rhodotorula toruloides* to mice by subcutaneous injection or oral administration to reduce plasma phenylalanine levels. However, phenylalanine lyases derived from microbial or plant sources become rapidly inactivated upon introduction into the human body due to immune responses, and are unstable in the human body, being readily degraded by proteases, leading to loss of activity.

In therapeutic studies using PKU model mice, four wild-type PAL proteins from different *sources-Rhodotorula toruloides* (R.h. PAL), *Nostoc punctiforme* (N.p. PAL), *Phormidium ceylanicum* (P.c. PAL), and *Anabaena variabilis* (A.v. PAL)-were tested. Among them, R.h. PAL and A.v. PAL as well as their PEGylated conjugates were considered more suitable for the treatment of PKU mice due to their highest specific enzyme activity and advantages in optimal reaction pH, Km parameter, protease resistance, and thermal stability, which is presumed to be related to the number and molecular characteristics of amino acids in their wild-type sequences.

Currently, results from phase III clinical studies of the marketed pegylated phenylalanine lyase drug PEGVALIASE show that among 261 subjects treated with pegylated phenylalanine lyase, 72.0% and 32.6% reached study endpoints of $\geq$12 months and $\geq$24 months, respectively, and 65% of the subjects are still receiving treatment. Within 24 months, 68.4% of participants achieved blood Phe $\leq$ 600 $\mu$mol/L, 60.7% achieved blood Phe $\leq$ 360 $\mu$mol/L, and 51.2% achieved blood Phe $\leq$ 120 $\mu$mol/L. During administration, serum phenylalanine levels were temporarily controlled effectively; however, as the number of administrations increased, the anti-PAL IgM/IgG antibody titers gradually rose, resulting in increased plasma drug clearance, necessitating continuous increases in administration dose and administration frequency to maintain efficacy.

However, in one aspect, the present disclosure provides a phenylalanine lyase mutant. According to an embodiment of the present disclosure, compared with an amino acid sequence of phenylalanine lyase, the phenylalanine lyase mutant has a mutation site at least one of site 72, site 240, site 467, and site 544. By introducing mutations at these specific sites of phenylalanine lyase, the resulting phenylalanine lyase mutant, when modified with polyethylene glycol, not only increases the average degree of modification but also improves enzyme activity retention rate, reduces immunogenicity, and extends plasma drug concentration maintenance to 5 to 7 days.

In another aspect, the present disclosure provides a nucleic acid molecule. According to an embodiment of the present disclosure, the nucleic acid molecule encodes the aforementioned phenylalanine lyase mutant. The nucleic acid molecule according to an embodiment of the present disclosure encodes the aforementioned phenylalanine lyase mutant.

In yet another aspect, the present disclosure provides an expression vector. According to an embodiment of the present disclosure, the expression vector carries the aforementioned nucleic acid molecule. The expression vector according to the embodiment of the present disclosure can effectively express the aforementioned phenylalanine lyase mutant, thereby enabling large-scale in vitro production of the phenylalanine lyase mutant.

In yet another aspect, the present disclosure provides a recombinant cell. According to an embodiment of the present disclosure, the recombinant cell carries the aforementioned nucleic acid molecule or the aforementioned expression vector, or expresses the aforementioned phenylalanine lyase mutant. Using the recombinant cell under suitable conditions, the phenylalanine lyase mutant described above can be efficiently expressed in the cell.

In yet another aspect of the present disclosure, a phenylalanine lyase mutant conjugate or a pharmaceutically acceptable salt thereof is provided. According to an embodiment of the present disclosure, the phenylalanine lyase mutant conjugate or a pharmaceutically acceptable salt thereof includes: the phenylalanine lyase mutant as described above; and a modifying group conjugated to the phenylalanine lyase mutant. The phenylalanine lyase mutant conjugate or a pharmaceutically acceptable salt thereof of the present disclosure has the advantages of high average degree of modification of polyethylene glycol, high enzyme activity retention rate, low immunogenicity, and long blood drug maintenance time.

In yet another aspect of the present disclosure, a polyethylene glycol-modified phenylalanine lyase mutant is provided. According to an embodiment of the present disclosure, the phenylalanine lyase mutant has PEG modification at at least the following amino acid positions: $K^{72}$, $K^{240}$, $K^{467}$, and $K^{544}$. The polyethylene glycol-modified phenylalanine lyase mutant of the present disclosure has the advantages of high average degree of modification, high enzyme activity retention rate, low immunogenicity, and long blood-drug maintenance time.

In yet another aspect of the present disclosure, a pharmaceutical composition is provided. According to an embodiment of the present disclosure, the pharmaceutical composition includes: the phenylalanine lyase mutant described above, or the phenylalanine lyase mutant conjugate or a pharmaceutically acceptable salt thereof described above. The pharmaceutical composition of the present disclosure is effective for preventing and/or treating hyperphenylalaninemia-related diseases, particularly the treatment of phenylketonuria.

In still another aspect of the present disclosure, there is provided use of the phenylalanine lyase mutant described above, the phenylalanine lyase mutant conjugate or a pharmaceutically acceptable salt thereof described above, the polyethylene glycol-modified phenylalanine lyase mutant described above, or the pharmaceutical composition described above in the manufacture of a medicament for preventing and/or treating a hyperphenylalaninemia-related disease.

In yet another aspect, the present disclosure provides a method for reducing immunogenicity while maintaining enzymatic activity of phenylalanine lyase. According to an embodiment of the present disclosure, the method includes:

performing PEG modification at a mutation site of a mutated phenylalanine lyase. The mutation site comprises at least one of E72K, Q240K, R467K, and R544K. The method of the present disclosure reduces the immunogenicity of phenylalanine lyase while maintaining a high enzyme activity retention rate.

Additional aspects and advantages of the present disclosure will be shown in part in the description which follows and, in part, will be obvious from the description, or may be learned by practice of the present disclosure.

## BRIEF DESCRIPTION OF DRAWINGS

The foregoing and/or additional aspects and advantages of the present disclosure will become apparent and more readily appreciated from the description of embodiments with reference to the accompanying drawings.

FIG. 1 shows the SDS-PAGE purity analysis results of the phenylalanine lyase mutant according to Example 2 of the present disclosure.

FIG. 2 shows the SEC-HPLC purity analysis results of the phenylalanine lyase mutant according to Example 2 of the present disclosure.

FIG. 3 shows the modification site analysis results of the site-specific conjugate of the phenylalanine lyase mutant according to Example 4 of the present disclosure.

FIG. 4 shows changes in blood drug concentration in rats after multiple administrations of polyethylene glycol conjugates of phenylalanine lyase and its mutants with different average degrees of modification according to Example 5 of the present disclosure.

FIG. 5 shows changes in serum titers of anti-PEG-IgM/IgG antibody (A) and anti-PAL-IgG antibody (B) in rats after multiple administrations of the polyethylene glycol conjugate of the phenylalanine lyase mutant according to Example 5 of the present disclosure.

FIG. 6 shows changes in serum phenylalanine concentration in Pah[enu] mice after multiple administrations of the polyethylene glycol conjugate of phenylalanine lyase mutant according to Example 7 at different doses.

FIG. 7 shows changes in plasma drug concentration in Pah[enu] mice after multiple administrations of the polyethylene glycol conjugate of phenylalanine lyase mutant according to Example 7 at different doses.

FIG. 8 shows anti-PEG IgG antibody titers in Pah[enu] mice after multiple administrations of the polyethylene glycol conjugate of phenylalanine lyase mutant according to Example 7 at different doses.

FIG. 9 shows anti-PEG IgM antibody titers in Pah[enu] mice after multiple administrations of the polyethylene glycol conjugate of phenylalanine lyase mutant according to Example 7 at different doses.

FIG. 10 shows the pharmacokinetic curve in Beagle dogs after a single administration of the polyethylene glycol conjugate of phenylalanine lyase mutant of the present disclosure according to Example 9.

FIG. 11 shows changes in serum phenylalanine concentration in Pegvaliase-Pah[enu] mice after multiple administrations of the polyethylene glycol conjugate of phenylalanine lyase mutant according to Example 10 of the present disclosure.

FIG. 12 shows anti-PEG IgG and IgM antibody titers in Pegvaliase-Pah[enu] mice after multiple administrations of the polyethylene glycol conjugate of phenylalanine lyase mutant according to Example 10 of the present disclosure.

## DESCRIPTION OF EMBODIMENTS

Hereinafter, embodiments of the present disclosure are described in detail. The embodiments described below are illustrative only and are not to be construed as limiting the present disclosure.

It should be noted that the terms "first" and "second" are used for descriptive purposes only and are not to be construed as indicating or implying relative importance or as implicitly indicating the number of technical features indicated. Thus, a feature defined by "first" or "second" may explicitly or implicitly include one or more such features. Further, in the description of the present disclosure, unless otherwise specified, the term "plurality" means two or more.

As used herein, the terms "include(s)" or "comprise(s)" are open-ended expressions, i.e., they include the features specified by the present disclosure but do not exclude other features.

As used herein, the terms "optionally, " "optional, " or "option" generally mean that the event or circumstance described subsequently may or may not occur, and the description includes both cases where the event or circumstance occurs and cases where it does not.

As used herein, the terms "identity", "homology", or "similarity", when describing an amino acid sequence or nucleic acid sequence relative to a reference sequence, refer to the percentage of identical amino acids or nucleotides between two amino acid sequences or nucleic acid sequences determined by conventional methods; for example, see Ausubel et al., eds. (1995), Current Protocols in Molecular Biology, Chapter 19 (Greene Publishing and Wiley-Interscience, New York); and the ALIGN program (Dayhoff (1978), Atlas of Protein Sequence and Structure 5: Suppl. 3 (National Biomedical Research Foundation, Washington, D.C.). Several algorithms are available for aligning sequences and determining sequence identity, including the homology alignment algorithm by Needleman et al. (1970) J. Mol. Biol. 48: 443; the local homology algorithm by Smith et al. (1981) Adv. Appl. Math. 2: 482; the similarity search method by Pearson et al. (1988) Proc. Natl. Acad. Sci. 85: 2444; Smith-Waterman algorithm (Meth. Mol. Biol. 70: 173-187(1997); and BLASTP, BLASTN, and BLASTX algorithms (see Altschul et al. (1990) J. Mol. Biol. 215: 403-410). Computer programs implementing these algorithms are also available and include, but are not limited to: ALIGN or Megalign (DNASTAR) software, or WU-BLAST-2 (Altschul et al., Meth. Enzym. 266: 460-480(1996)); or GAP, BESTFIT, BLAST Altschul et al., supra, FASTA, and TFASTA, available in the Genetics Computing Group (GCG) package, Version 8, Madison, Wisconsin, USA; and CLUSTAL in the PC/Gene program provided by Intelligenetics, Mountain View, California.

As used herein, the term "at least 50% homology" refers to homology of at least 50% with each reference sequence and may be 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.5%, or 99.9%. The term "at least 90% homology" refers to homology of at least 90% with each reference sequence and may be 90%, 91%, 92%, 93%, 94%, 95%, 96%, 97%, 98%, 99%, 99.5%, 99.9%.

As used herein, the term "pharmaceutical composition" generally refers to a unit dose form and may be prepared by any of the methods well known in the art of pharmacy. All methods include the step of bringing the active ingredient into association with a carrier, which constitutes one or more accessory ingredients. In general, pharmaceutical compositions are prepared by uniformly and intimately bringing into association an active phenylalanine lyase mutant, a phenylalanine lyase mutant conjugate, or a pharmaceutically acceptable salt thereof with liquid carriers, finely divided solid carriers, or both.

As used herein, the term "pharmaceutically acceptable excipient" includes any solvent, solid excipient, diluent, or other liquid excipient, etc., suitable for the particular dose form desired. Except for conventional excipients that are incompatible with the phenylalanine lyase mutant, phenylalanine lyase mutant conjugate, or pharmaceutically acceptable salt thereof of the present disclosure, such as those causing undesirable biological effects or deleterious interactions with any other component(s) of the pharmaceutical composition, their uses are contemplated to be within the scope of the present disclosure.

As used herein, the term "administering" refers to introducing a predetermined amount of a substance into a patient by a suitable method. The phenylalanine lyase mutant, the phenylalanine lyase mutant conjugate or a pharmaceutically acceptable salt thereof, or the pharmaceutical composition of the present disclosure can be administered via any common route, provided it can reach the target tissue. Various routes of administration are contemplated, including intraperitoneal, intravenous injection, intramuscular injection, subcutaneous injection, and the like, although the present disclosure is not limited to these exemplary routes. Preferably, the phenylalanine lyase mutant, the phenylalanine lyase mutant conjugate or a pharmaceutically acceptable salt thereof, or the pharmaceutical composition of the present disclosure is administered by intravenous injection or subcutaneous injection.

As used herein, the term "treat" refers to achieving a desired pharmacological and/or physiological effect. The effect may be prophylactic in terms of completely or partially preventing a disease or its symptoms, and/or therapeutic in terms of partially or completely curing a disease and/or adverse effects caused by the disease. As used herein, "treat" refers to treatment of a disease in a mammal, particularly a human, and includes: (a) prevention of the disease or condition in an individual who may be predisposed to the disease but has not yet been diagnosed as having it; (b) inhibiting the disease, for example, arresting its development; or (c) relieving the disease, e.g., alleviating symptoms associated with the disease. As used herein, "treat" encompasses any administration of a phenylalanine lyase mutant, a phenylalanine lyase mutant conjugate or a pharmaceutically acceptable salt thereof, a pharmaceutical composition, or a medicament to a subject to treat, cure, alleviate, ameliorate, lessen, or inhibit a disease in the subject, including but not limited to administration of a pharmaceutical agent as described herein to a subject in need thereof.

As used herein, the term "cancer" or "tumor" refers to any unregulated cell growth. Illustratively, it may be non-small cell lung cancer, papillary thyroid carcinoma, glioblastoma multiforme, colon cancer, rectal cancer, lung cancer, head and neck cancer, kidney cancer, bladder cancer, breast cancer, ovarian cancer, liver cancer, bile duct cancer, sarcoma, acute myeloid leukemia, large cell neuroendocrine carcinoma, neuroblastoma, prostate cancer, neuroblastoma, pancreatic cancer, melanoma, head and neck squamous cell carcinoma, cervical cancer, skin cancer, glioma, esophageal cancer, oral squamous cell carcinoma, or gastric cancer, and the like.

The present disclosure provides a phenylalanine lyase mutant, a nucleic acid molecule, an expression vector, a recombinant cell, a phenylalanine lyase mutant conjugate or a pharmaceutically acceptable salt thereof, a polyethylene glycol-modified phenylalanine lyase mutant, a pharmaceutical composition, uses thereof, and a method for reducing immunogenicity while maintaining enzymatic activity of phenylalanine lyase, each of which is described in detail below.

**Phenylalanine lyase mutant**

In one aspect of the present disclosure, there is provided a phenylalanine lyase mutant. According to an embodiment of the present disclosure, compared with an amino acid sequence of phenylalanine lyase, the phenylalanine lyase mutant has a mutation site at least one of site 72, site 240, site 467, and site 544. By introducing mutations at these specific sites of phenylalanine lyase, the resulting phenylalanine lyase mutant, when modified with polyethylene glycol, not only increases the average degree of modification but also improves enzyme activity retention rate, reduces immunogenicity, and extends plasma drug concentration maintenance to 5 to 7 days.

According to an embodiment of the present disclosure, the above-mentioned phenylalanine lyase mutant may further include at least one of the following technical features:

According to an embodiment of the present disclosure, the mutation site is defined with reference to an amino acid sequence as set forth in SEQ ID NO: 1.

MKTLSQAQSKTSSQQFSFTGNSSANVIIGNQKLTINDVARVARNGTLVSLTN

NTDILQGIQASCDYINNAVESGEPIYGVTSGFGGMANVAISREQASELQTNLVWFLKT

GAGNKLPLADVRAAMLLRANSHMRGASGIRLELIKRMEIFLNAGVTPYVYEFGSIGA

SGDLVPLSYITGSLIGLDPSFKVDFNGKEMDAPTALRQLNLSPLTLLPKEGLAMMNGT

SVMTGIAANCVYDTQILTAIAMGVHALDIQALNGTNQSFHPFIHNSKPHPGQLWAAD

QMISLLANSQLVRDELDGKHDYRDHELIQDRYSLRCLPQYLGPIVDGISQIAKQIEIEIN

SVTDNPLIDVDNQASYHGGNFLGQYVGMGMDHLRYYIGLLAKHLDVQIALLASPEFS

NGLPPSLLGNRERKVNMGLKGLQICGNSIMPLLTFYGNSIADRFPTHAEQFNQNINSQ

GYTSATLARRSVDIFQNYVAIALMFGVQAVDLRTYKKTGHYDARASLSPATERLYSAV

RHVVGQKPTSDRPYIWNDNEQGLDEHIARISADIA AGGVIVQAVQ DILPSLH (SEQ ID

NO: 1)

According to an embodiment of the present disclosure, the phenylalanine lyase has an amino acid sequence as set forth in SEQ ID NO: 2 to SEQ ID NO: 3, or an amino acid sequence having at least 50% homology thereto.

MKTLSQAQSKTSSQQFSFTGNSSANVIIGNQKLTINDVARVARNGTLVSLTN

NTDILQGIQASCDYINNAVESGEPIYGVTSGFGGMANVAISREQASELQTNLVWFLKT

GAGNKLPLADVRAAMLLRANSHMRGASGIRLELIKRMEIFLNAGVTPYVYEFGSIGA

SGDLVPLSYITGSLIGLDPSFKVDFNGKEMDAPTALRQLNLSPLTLLPKEGLAMMNGT

SVMTGIAANCVYDTQILTAIAMGVHALDIQALNGTNQSFHPFIHNSKPHPGQLWAAD

QMISLLANSQLVRDELDGKHDYRDHELIQDRYSLRCLPQYLGPIVDGISQIAKQIEIEIN

SVTDNPLIDVDNQASYHGGNFLGQYVGMGMDHLRYYIGLLAKHLDVQIALLASPEFS

NGLPPSLLGNRERKVNMGLKGLQICGNSIMPLLTFYGNSIADRFPTHAEQFNQNINSQ

GYTSATLARRSVDIFQNYVAIALMFGVQAVDLRTYKKTGHYDARACLSPATERLYSAV

RHVVGQKPTSDRPYIWNDNEQGLDEHIARISADIA AGGVIVQAVQ DILPCLH (SEQ ID

NO: 2)

MNITSLQQNITRSWQIPFTNSSDSIVTVGDRNLTIDEVVNVARHGTQVRLTD

NADVIRGVQASCDYINNAVETAQPIYGVTSGFGGMADVVISREQAAELQTNLIWFLKS

GAGNKLSLADVRAAMLLRANSHLYGASGIRLELIQRIETFLNAGVTPHVYEFGSIGAS

GDLVPLSYITGALIGLDPSFTVDFDGKEMDAVTALSRLGLPKLQLQPKEGLAMMNGT

SVMTGIAANCVYDAKVLLALTMGVHALAIQGLYGTNQSFHPFIHQCKPHPGQLWTA

DQMFSLLKDSSLVREELDGKHEYRGKDLIQDRYSLRCLAQFIGPIVDGVSEITKQIEVE

MNSVTDNPLIDVENQVSYHGGNFLGQYVGVTMDRLRYYIGLLAKHIDVQIALLVSPE

FSNGLPPSLVGNSDRKVNMGLKGLQISGNSIMPLLSFYGNSLADRFPTHAEQFNQNIN

SQGYISANLTRRSVDIFQNYMAIALMFGVQAVDLRTYKMKGHYDARTCLSPNTVQLY

TAVCEVVGKPLTSVRPYIWNDNEQCLDEHIARISADIAGGGLIVQAVEHIFSSLKST

(SEQ ID NO: 3)

Among them, the amino acid sequences as set forth in SEQ ID NO: 1 and SEQ ID NO: 2 are phenylalanine lyase derived from *Anabaena variabilis* or a variant thereof, and the amino acid sequence as set forth in SEQ ID NO: 3 is phenylalanine lyase derived from *Nostoc punctiformis.*

Note that the mutation site of the present disclosure is defined with reference to the amino acid sequence as set forth in SEQ ID NO: 1. For example, site 72 refers to the amino acid at site 72 based on the amino acid sequence as set forth in SEQ ID NO: 1; site 240 refers to the amino acid at site 240 based on the amino acid sequence as set forth in SEQ ID NO: 1; site 467 refers to the amino acid at site 467 based on the amino acid sequence as set forth in SEQ ID NO: 1; site 544 refers to the amino acid at site 544 based on the amino acid sequence as set forth in SEQ ID NO: 1. The mutation sites of the amino acid sequences as set forth in SEQ ID NO: 2 to SEQ ID NO: 3 can be determined based on homology comparison with SEQ ID NO: 1; for example, homology comparison may be performed using the homology alignment algorithm by Needleman et al. (1970) J. Mol. Biol. 48: 443 or the local homology algorithm by Smith et al. (1981) Adv. Appl. Math. 2: 482.

Illustratively, compared with an amino acid sequence as set forth in SEQ ID NO: 1, the phenylalanine lyase mutant has a mutation site at least one of site 72, site 240, site 467, and site 544.

Illustratively, compared with an amino acid sequence as set forth in SEQ ID NO: 2, the phenylalanine lyase mutant has a mutation site at least one of site 72, site 240, site 467, and site 544.

Illustratively, compared with an amino acid sequence as set forth in SEQ ID NO: 3, the phenylalanine lyase mutant has a mutation site at least one of site 72, site 240, site 467, and site 544.

According to an embodiment of the present disclosure, the mutation site of the phenylalanine lyase mutant corresponds to an amino acid capable of conjugating with a modifying group.

According to an embodiment of the present disclosure, the amino acid capable of conjugating with a modifying group is lysine.

According to an embodiment of the present disclosure, the mutation site of the phenylalanine lyase mutant corresponds to an amino acid having an ASA (accessible surface area) value of ≥ 60% or an RSA (relative solvent accessibility) value of ≥ 30%.

According to an embodiment of the present disclosure, the mutation site of the phenylalanine lyase mutant corresponds to an amino acid having a pKa value > 9.0.

According to an embodiment of the present disclosure, compared with an amino acid sequence as set forth in SEQ ID NO: 1 or SEQ ID NO: 2, the phenylalanine lyase mutant includes at least one mutation site of E72K, Q240K, R467K, and R544K. Thus, for the phenylalanine lyase mutant, the average degree of modification by polyethylene glycol and enzyme activity retention rate can be improved and immunogenicity can be reduced.

It is noted that "includes at least one mutation site of E72K, Q240K, R467K, and R544K" means that, in addition to at least one of the above four mutation sites, the phenylalanine lyase mutant of the present disclosure may further include other mutation sites.

According to an embodiment of the present disclosure, compared with an amino acid sequence as set forth in SEQ ID NO: 3, the phenylalanine lyase mutant comprises at least one mutation site of E72K, R467K, and R544K. Thus, for the phenylalanine lyase mutant, the average degree of modification by polyethylene glycol and enzyme activity retention rate can be improved and immunogenicity can be reduced.

According to an embodiment of the present disclosure, compared with an amino acid sequence as set forth in SEQ ID NO: 1 or SEQ ID NO: 2, the phenylalanine lyase mutant includes: 1) mutation sites of R467K and R544K; 2) mutation sites of E72K, R467K, and R544K; or 3) mutation sites of E72K, Q240K, R467K, and R544K. Thus, for the phenylalanine lyase mutant, the average degree of modification by polyethylene glycol and enzyme activity retention rate can be improved and immunogenicity can be reduced.

According to an embodiment of the present disclosure, compared with an amino acid sequence as set forth in SEQ ID NO: 3, the phenylalanine lyase mutant includes: 1) mutation sites of R467K and R544K; or 2) mutation sites of E72K, R467K, and R544K

According to an embodiment of the present disclosure, the phenylalanine lyase mutant has an amino acid sequence as set forth in any one of SEQ ID NO: 4 to SEQ ID NO: 11, or an amino acid sequence having at least 50% homology thereto.

MKTLSQAQSKTSSQQFSFTGNSSANVIIGNQKLTINDVARVARNGTLVSLTN
NTDILQGIQASCDYINNAVESGEPIYGVTSGFGGMANVAISREQASELQTNLVWFLKT
GAGNKLPLADVRAAMLLRANSHMRGASGIRLELIKRMEIFLNAGVTPYVYEFGSIGA
SGDLVPLSYITGSLIGLDPSFKVDFNGKEMDAPTALRQLNLSPLTLLPKEGLAMMNGT
SVMTGIAANCVYDTQILTAIAMGVHALDIQALNGTNQSFHPFIHNSKPHPGQLWAAD
QMISLLANSQLVRDELDGKHDYRDHELIQDRYSLRCLPQYLGPIVDGISQIAKQIEIEIN
SVTDNPLIDVDNQASYHGGNFLGQYVGMGMDHLRYYIGLLAKHLDVQIALLASPEFS
NGLPPSLLGNRERKVNMGLKGLQICGNSIMPLLTFYGNSIADRFPTHAEQFNQNINSQ
GYTSATLARKSVDIFQNYVAIALMFGVQAVDLRTYKKTGHYDARASLSPATERLYSAV
RHVVGQKPTSDRPYIWNDNEQGLDEHIAKISADIAAGGVIVQAVQDILPSLH (SEQ ID NO: 4)

MKTLSQAQSKTSSQQFSFTGNSSANVIIGNQKLTINDVARVARNGTLVSLTN
NTDILQGIQASCDYINNAVKSGEPIYGVTSGFGGMANVAISREQASELQTNLVWFLKT
GAGNKLPLADVRAAMLLRANSHMRGASGIRLELIKRMEIFLNAGVTPYVYEFGSIGA
SGDLVPLSYITGSLIGLDPSFKVDFNGKEMDAPTALRQLNLSPLTLLPKEGLAMMNGT
SVMTGIAANCVYDTQILTAIAMGVHALDIQALNGTNQSFHPFIHNSKPHPGQLWAAD
QMISLLANSQLVRDELDGKHDYRDHELIQDRYSLRCLPQYLGPIVDGISQIAKQIEIEIN
SVTDNPLIDVDNQASYHGGNFLGQYVGMGMDHLRYYIGLLAKHLDVQIALLASPEFS
NGLPPSLLGNRERKVNMGLKGLQICGNSIMPLLTFYGNSIADRFPTHAEQFNQNINSQ
GYTSATLARKSVDIFQNYVAIALMFGVQAVDLRTYKKTGHYDARASLSPATERLYSAV
RHVVGQKPTSDRPYIWNDNEQGLDEHIAKISADIAAGGVIVQAVQDILPSLH (SEQ ID NO: 5)

MKTLSQAQSKTSSQQFSFTGNSSANVIIGNQKLTINDVARVARNGTLVSLTN NTDILQGIQASCDYINNAVKSGEPIYGVTSGFGGMANVAISREQASELQTNLVWFLKT GAGNKLPLADVRAAMLLRANSHMRGASGIRLELIKRMEIFLNAGVTPYVYEFGSIGA SGDLVPLSYITGSLIGLDPSFKVDFNGKEMDAPTALRQLNLSPLTLLPKEGLAMMNGT SVMTGIAANCVYDTKILTAIAMGVHALDIQALNGTNQSFHPFIHNSKPHPGQLWAAD QMISLLANSQLVRDELDGKHDYRDHELIQDRYSLRCLPQYLGPIVDGISQIAKQIEIEIN SVTDNPLIDVDNQASYHGGNFLGQYVGMGMDHLRYYIGLLAKHLDVQIALLASPEFS NGLPPSLLGNRERKVNMGLKGLQICGNSIMPLLTFYGNSIADRFPTHAEQFNQNINSQ GYTSATLARKSVDIFQNYVAIALMFGVQAVDLRTYKKTGHYDARASLSPATERLYSAV RHVVGQKPTSDRPYIWNDNEQGLDEHIAKISADIA AGGVIVQAVQ DILPSLH (SEQ ID NO: 6)

MKTLSQAQSKTSSQQFSFTGNSSANVIIGNQKLTINDVARVARNGTLVSLTN NTDILQGIQASCDYINNAVESGEPIYGVTSGFGGMANVAISREQASELQTNLVWFLKT GAGNKLPLADVRAAMLLRANSHMRGASGIRLELIKRMEIFLNAGVTPYVYEFGSIGA SGDLVPLSYITGSLIGLDPSFKVDFNGKEMDAPTALRQLNLSPLTLLPKEGLAMMNGT SVMTGIAANCVYDTQILTAIAMGVHALDIQALNGTNQSFHPFIHNSKPHPGQLWAAD QMISLLANSQLVRDELDGKHDYRDHELIQDRYSLRCLPQYLGPIVDGISQIAKQIEIEIN SVTDNPLIDVDNQASYHGGNFLGQYVGMGMDHLRYYIGLLAKHLDVQIALLASPEFS NGLPPSLLGNRERKVNMGLKGLQICGNSIMPLLTFYGNSIADRFPTHAEQFNQNINSQ GYTSATLARKSVDIFQNYVAIALMFGVQAVDLRTYKKTGHYDARACLSPATERLYSAV RHVVGQKPTSDRPYIWNDNEQGLDEHIAKISAIA AGGVIVQAVQ DILPCLH (SEQ ID NO: 7)

MKTLSQAQSKTSSQQFSFTGNSSANVIIGNQKLTINDVARVARNGTLVSLTN NTDILQGIQASCDYINNAVKSGEPIYGVTSGFGGMANVAISREQASELQTNLVWFLKT GAGNKLPLADVRAAMLLRANSHMRGASGIRLELIKRMEIFLNAGVTPYVYEFGSIGA SGDLVPLSYITGSLIGLDPSFKVDFNGKEMDAPTALRQLNLSPLTLLPKEGLAMMNGT SVMTGIAANCVYDTQILTAIAMGVHALDIQALNGTNQSFHPFIHNSKPHPGQLWAAD QMISLLANSQLVRDELDGKHDYRDHELIQDRYSLRCLPQYLGPIVDGISQIAKQIEIEIN SVTDNPLIDVDNQASYHGGNFLGQYVGMGMDHLRYYIGLLAKHLDVQIALLASPEFS NGLPPSLLGNRERKVNMGLKGLQICGNSIMPLLTFYGNSIADRFPTHAEQFNQNINSQ GYTSATLARKSVDIFQNYVAIALMFGVQAVDLRTYKKTGHYDARACLSPATERLYSAV RHVVGQKPTSDRPYIWNDNEQGLDEHIAKISAIA AGGVIVQAVQ DILPCLH (SEQ ID NO:8)

MKTLSQAQSKTSSQQFSFTGNSSANVIIGNQKLTINDVARVARNGTLVSLTN NTDILQGIQASCDYINNAVKSGEPIYGVTSGFGGMANVAISREQASELQTNLVWFLKT GAGNKLPLADVRAAMLLRANSHMRGASGIRLELIKRMEIFLNAGVTPYVYEFGSIGA SGDLVPLSYITGSLIGLDPSFKVDFNGKEMDAPTALRQLNLSPLTLLPKEGLAMMNGT SVMTGIAANCVYDTKILTAIAMGVHALDIQALNGTNQSFHPFIHNSKPHPGQLWAAD QMISLLANSQLVRDELDGKHDYRDHELIQDRYSLRCLPQYLGPIVDGISQIAKQIEIEIN SVTDNPLIDVDNQASYHGGNFLGQYVGMGMDHLRYYIGLLAKHLDVQIALLASPEFS NGLPPSLLGNRERKVNMGLKGLQICGNSIMPLLTFYGNSIADRFPTHAEQFNQNINSQ GYTSATLARKSVDIFQNYVAIALMFGVQAVDLRTYKKTGHYDARACLSPATERLYSAV RHVVGQKPTSDRPYIWNDNEQGLDEHIAKISAIA AGGVIVQAVQ DILPCLH (SEQ ID NO: 9)

MNITSLQQNITRSWQIPFTNSSDSIVTVGDRNLTIDEVVNVARHGTQVRLTD NADVIRGVQASCDYINNAVETAQPIYGVTSGFGGMADVVISREQAAELQTNLIWFLKS GAGNKLSLADVRAAMLLRANSHLYGASGIRLELIQRIETFLNAGVTPHVYEFGSIGAS GDLVPLSYITGALIGLDPSFTVDFDGKEMDAVTALSRLGLPKLQLQPKEGLAMMNGT SVMTGIAANCVYDAKVLLALTMGVHALAIQGLYGTNQSFHPFIHQCKPHPGQLWTA

DQMFSLLKDSSLVREELDGKHEYRGKDLIQDRYSLRCLAQFIGPIVDGVSEITKQIEVE

MNSVTDNPLIDVENQVSYHGGNFLGQYVGVTMDRLRYYIGLLAKHIDVQIALLVSPE

FSNGLPPSLVGNSDRKVNMGLKGLQISGNSIMPLLSFYGNSLADRFPTHAEQFNQNIN

SQGYISANLTRKSVDIFQNYMAIALMFGVQAVDLRTYKMKGHYDARTCLSPNTVQLY

TAVCEVVGKPLTSVRPYIWNDNEQCLDEHIAKISADIAGGGLIVQAVEHIFSSLKST

(SEQ ID NO:10)

MNITSLQQNITRSWQIPFTNSSDSIVTVGDRNLTIDEVVNVARHGTQVRLTD

NADVIRGVQASCDYINNAVKTAQPIYGVTSGFGGMADVVISREQAAELQTNLIWFLK

SGAGNKLSLADVRAAMLLRANSHLYGASGIRLELIQRIETFLNAGVTPHVYEFGSIGA

SGDLVPLSYITGALIGLDPSFTVDFDGKEMDAVTALSRLGLPKLQLQPKEGLAMMNG

TSVMTGIAANCVYDAKVLLALTMGVHALAIQGLYGTNQSFHPFIHQCKPHPGQLWTA

DQMFSLLKDSSLVREELDGKHEYRGKDLIQDRYSLRCLAQFIGPIVDGVSEITKQIEVE

MNSVTDNPLIDVENQVSYHGGNFLGQYVGVTMDRLRYYIGLLAKHIDVQIALLVSPE

FSNGLPPSLVGNSDRKVNMGLKGLQISGNSIMPLLSFYGNSLADRFPTHAEQFNQNIN

SQGYISANLTRKSVDIFQNYMAIALMFGVQAVDLRTYKMKGHYDARTCLSPNTVQLY

TAVCEVVGKPLTSVRPYIWNDNEQCLDEHIAKISADIAGGGLIVQAVEHIFSSLKST

(SEQ ID NO: 11)

**Nucleic acid molecule, expression vector, and recombinant cell**

In another aspect, the present disclosure provides a nucleic acid molecule. According to an embodiment of the present disclosure, the nucleic acid molecule encodes the aforementioned phenylalanine lyase mutant. The nucleic acid molecule according to an embodiment of the present disclosure encodes the aforementioned phenylalanine lyase mutant.

According to an embodiment of the present disclosure, the nucleic acid molecule is DNA.

It should be noted that for the nucleic acid molecules referred to in the present disclosure, those skilled in the art will understand that either one or both strands of the complementary double-stranded nucleic acid are included. For convenience, in the present disclosure, although only one strand is typically shown, the complementary strand is also disclosed. In addition, the nucleic acid sequences of the present disclosure include DNA forms and RNA forms; the disclosure of one form implies the disclosure of the other.

In yet another aspect, the present disclosure provides an expression vector. According to an embodiment of the present disclosure, the expression vector carries the aforementioned nucleic acid molecule. When the nucleic acid molecule is ligated to the vector, the nucleic acid molecule may be linked directly or indirectly to control elements on the vector, as long as such control elements can regulate translation and expression of the nucleic acid molecule. These control elements may be derived directly from the vector itself, or they may be exogenous, i.e., not derived from the vector itself. It is, of course, sufficient that the nucleic acid molecule is operably linked to a control element. As used herein, "operably linked" refers to linking a foreign gene to a vector such that control elements within the vector, e.g., transcription control sequences and translation control sequences, etc., are capable of performing their intended function of regulating the transcription and translation of the foreign gene. Commonly used vectors may be, for example, plasmids, bacteriophages, and the like. The expression vector, according to some specific embodiments of the present disclosure, when introduced into a suitable recipient cell, can effectively achieve the expression of the phenylalanine lyase mutant as described above under the mediation of a regulatory system, thereby achieving mass production of the phenylalanine lyase mutant in vitro.

According to an embodiment of the present disclosure, the expression vector is a eukaryotic expression vector, a prokaryotic expression vector, a virus, or a bacteriophage.

According to an embodiment of the present disclosure, the expression vector is a lentiviral expression vector.

In yet another aspect, the present disclosure provides a recombinant cell. According to an embodiment of the present disclosure, the recombinant cell carries the aforementioned nucleic acid molecule or the aforementioned expression vector, or expresses the aforementioned phenylalanine lyase mutant. Using the recombinant cell under suitable conditions, the phenylalanine lyase mutant described above can be efficiently expressed in the cell.

According to an embodiment of the present disclosure, the recombinant cell is obtained by introducing the aforementioned expression vector into a host cell.

**Phenylalanine lyase mutant conjugate or pharmaceutically acceptable salt thereof**

In yet another aspect of the present disclosure, a phenylalanine lyase mutant conjugate or a pharmaceutically acceptable salt thereof is provided. According to an embodiment of the present disclosure, the phenylalanine lyase mutant conjugate or a pharmaceutically acceptable salt thereof includes: the phenylalanine lyase mutant as described above; and a modifying group conjugated to the phenylalanine lyase mutant. The phenylalanine lyase mutant conjugate or a pharmaceutically acceptable salt thereof of the present disclosure has the advantages of high average degree of modification of polyethylene glycol, high enzyme activity retention rate, low immunogenicity, and long blood drug maintenance time.

According to an embodiment of the present disclosure, the modifying group is polyethylene glycol.

According to an embodiment of the present disclosure, the polyethylene glycol has a molecular weight between 2 and 40 KDa, such as between 2 and 10 KDa or between 5 and 20 KDa. As a result, the antibody titer after multiple administrations can be increased.

According to an embodiment of the present disclosure, the polyethylene glycol is a linear polyethylene glycol or a branched polyethylene glycol.

According to an embodiment of the present disclosure, the polyethylene glycol has a monomethoxy group or a hydroxyl group.

According to an embodiment of the present disclosure, the polyethylene glycol is a polyethylene glycol having a reactive group.

According to an embodiment of the present disclosure, the reactive group of the polyethylene glycol includes at least one of succinimidyl butyrate, succinimidyl propionate, succinimidyl acetate, succinimidyl carbonate, and nitrophenyl carbonate, preferably succinimidyl propionate.

According to an embodiment of the present disclosure, the modifying group is conjugated to a lysine residue of the phenylalanine lyase mutant via an amide bond.

According to an embodiment of the present disclosure, a conjugation proportion of lysine modified with the modifying group in the phenylalanine lyase mutant conjugate or the pharmaceutically acceptable salt thereof is not less than 50%.

According to an embodiment of the present disclosure, a conjugation proportion of lysine modified with the modifying group at the mutation site in the phenylalanine lyase mutant conjugate or the pharmaceutically acceptable salt thereof is not less than 80%.

According to an embodiment of the present disclosure, a molar ratio of the modifying group to the phenylalanine lyase mutant is (5 to 20):1, such as 5:1, 6:1, 7:1, 8:1, 9:1, 10:1, 11:1, 12:1, 13:1, 14:1, 15:1, 16:1, 17:1, 18:1, 19:1, and 20:1, and ranges between any two molar ratios, such as 8:1 to 20:1.

According to an embodiment of the present disclosure, a peptide map of the phenylalanine lyase mutant conjugate exhibits a peptide fragment with decreased peak area as shown in Table 4.

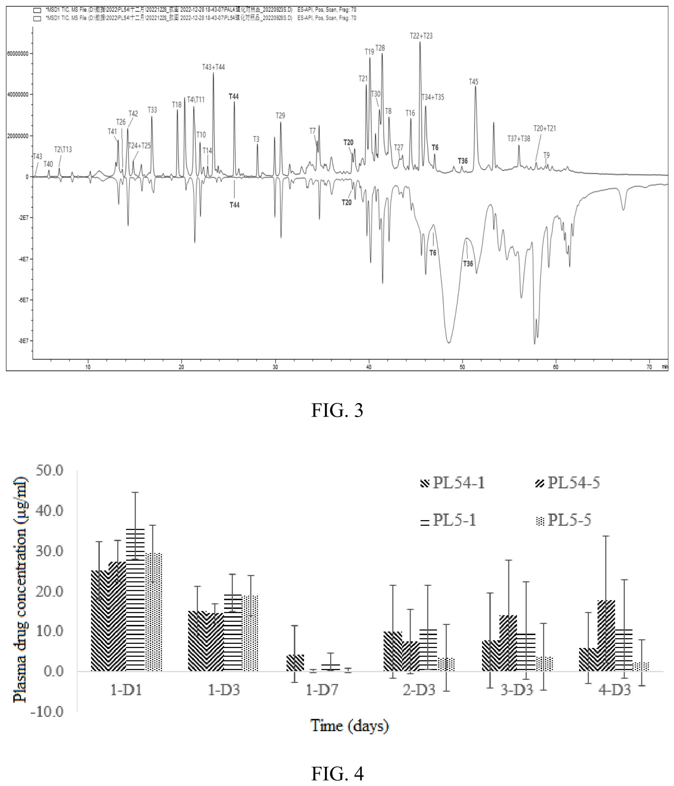

According to an embodiment of the present disclosure, a peptide map of the phenylalanine lyase mutant conjugate is as shown in FIG. 3.

**Polyethylene glycol-modified phenylalanine lyase mutant and preparation method thereof**

In yet another aspect of the present disclosure, a polyethylene glycol-modified phenylalanine lyase mutant is provided. According to an embodiment of the present disclosure, the phenylalanine lyase mutant has a PEG modification at at least the following amino acid positions: $K^{72}$, $K^{240}$, $K^{467}$, and $K^{544}$. The polyethylene glycol-modified phenylalanine lyase mutant of the present disclosure has the advantages of high average degree of modification, high enzyme activity retention rate, low immunogenicity, and long blood-drug maintenance time.

As used herein, "$K^{72}$" refers to a phenylalanine lyase mutant in which the amino acid site for PEG modification is lysine at site 72 of the phenylalanine lyase mutant.

According to an embodiment of the present disclosure, the phenylalanine lyase mutant is the phenylalanine lyase mutant as defined above.

According to an embodiment of the present disclosure, the phenylalanine lyase mutant has an amino acid sequence as set forth in any one of SEQ ID NO: 4 to SEQ ID NO: 11, or an amino acid sequence having at least 50% homology thereto.

Note that the mutation site in the present application is defined with reference to the amino acid sequences as set forth in SEQ ID NO: 1 and SEQ ID NO: 6. For example, site 72 refers to the amino acid at site 72 based on the amino acid sequence as set forth in SEQ ID NO: 6; site 240 refers to the amino acid at site 240 based on the amino acid sequence as set forth in SEQ ID NO: 6; site 467 refers to the amino acid at site 467 based on the amino acid sequence as set forth in SEQ ID NO: 6; site 544 refers to the amino acid at site 544 based on the amino acid sequence as set forth in SEQ ID NO: 6. The mutation sites of the amino acid sequences as set forth in in SEQ ID NO: 4, SEQ ID NO: 5, and SEQ ID NO: 7 to SEQ ID NO: 11 can be determined based on homology comparison with SEQ ID NO: 6; for example, homology comparison can be determined by the homology alignment algorithm by Needleman et al. (1970) J. Mol. Biol. 48: 443, the local homology algorithm by Smith et al. (1981) Adv. Appl. Math. 2: 482, or the like.

According to an embodiment of the present disclosure, $K^{467}$ and $K^{544}$ in the phenylalanine lyase mutant as set forth in SEQ ID NO: 4 are PEG-modified.

According to an embodiment of the present disclosure, $K^{72}$, $K^{467}$, and $K^{544}$ in the phenylalanine lyase mutant as set forth in SEQ ID NO: 5 are PEG-modified.

According to an embodiment of the present disclosure, $K^{72}$, $K^{240}$, $K^{467}$, and $K^{544}$ in the phenylalanine lyase mutant as set forth in SEQ ID NO: 6 are PEG-modified.

According to an embodiment of the present disclosure, $K^{467}$ and $K^{544}$ in the phenylalanine lyase mutant as set forth in SEQ ID NO: 7 are PEG-modified.

According to an embodiment of the present disclosure, $K^{72}$, $K^{467}$, and $K^{544}$ in the phenylalanine lyase mutant as set forth in SEQ ID NO: 8 are PEG-modified.

According to an embodiment of the present disclosure, $K^{72}$, $K^{240}$, $K^{467}$, and $K^{544}$ in the phenylalanine lyase mutant as set forth in SEQ ID NO: 9 are PEG-modified.

According to an embodiment of the present disclosure, $K^{467}$ and $K^{544}$ in the phenylalanine lyase mutant as set forth in SEQ ID NO: 10 are PEG-modified.

According to an embodiment of the present disclosure, $K^{72}$, $K^{467}$, and $K^{544}$ in the phenylalanine lyase mutant as set forth in SEQ ID NO: 11 are PEG-modified.

In a preferred embodiment of the present disclosure, the phenylalanine lyase mutant has an amino acid sequence as set forth in either SEQ ID NO: 6 or SEQ ID NO: 8.

As used herein, a polyethylene glycol-modified phenylalanine lyase mutant of the present disclosure refers to a phenylalanine lyase mutant obtained by covalently modifying a phenylalanine lyase with polyethylene glycol. The term " polyethylene glycol (PEG)" refers to a mixture of a condensation polymer of ethylene oxide and water, represented by the general formula $H(OCH_2CH_2)_nOH$, which is a pH-neutral, non-toxic, highly water-soluble hydrophilic polymer having a linear or branched structure. Due to its non-toxicity and good biocompatibility, the FDA has approved a variety of PEG-modified recombinant protein drugs for marketing, demonstrating that PEG can be used to reduce protein immunogenicity, increase protein solubility, and prolong protein half-life. Activation of one or more ends of PEG is required for conjugation of PEG to a protein, and a corresponding modifying group can be selected for activation depending on functional groups present on the target protein, such as amino, sulfhydryl, carboxyl, or hydroxyl groups.

In some alternative embodiments of the present disclosure, the site for PEG modification in the phenylalanine lyase mutant of the present disclosure is the ε-amino group of a lysine residue, although there is also minor modification of the α-amino group at the N-terminal lysine residue. The phenylalanine lyase mutant is covalently linked to a modifying group of PEG via an aliphatic amine bond, secondary amine bond, or amide bond; preferably, the polyethylene glycol molecule is conjugated to the phenylalanine lyase mutant to form an amide bond. The modifying groups of polyethylene glycol include, but are not limited to, N-hydroxysuccinimides, such as N-hydroxysuccinimide (NHS), N-hydroxysuccinimidyl carbonate (SC), N-hydroxysuccinimidyl acetate (SCM), N-hydroxysuccinimidyl propionate (SPA), N-hydroxysuccinimidyl butyrate (SBA), and N-hydroxysuccinimidyl succinate (SS). The blocking groups of polyethylene glycol include, but are not limited to, monomethoxy group, ethoxy group, glucose, or galactose, with monomethoxy group being preferred.

According to an embodiment of the present disclosure, polyethylene glycol used for the PEG modification has a molecular weight between 2 and 40 KDa, for example, between 2 and 10 KDa or between 5 and 20 KDa.

According to an embodiment of the present disclosure, the polyethylene glycol is a linear polyethylene glycol or a branched polyethylene glycol.

According to an embodiment of the present disclosure, the polyethylene glycol has a monomethoxy group or a hydroxyl group.

According to an embodiment of the present disclosure, the polyethylene glycol is a polyethylene glycol having a reactive group.

According to an embodiment of the present disclosure, the polyethylene glycol is conjugated to a lysine residue of the phenylalanine lyase mutant via an amide bond.

According to an embodiment of the present disclosure, the reactive group of the polyethylene glycol includes at least one of succinimidyl butyrate, succinimidyl propionate, succinimidyl acetate, succinimidyl carbonate, and nitrophenyl carbonate, preferably succinimidyl propionate.

According to an embodiment of the present disclosure, the peptide map of the polyethylene glycol-modified phenylalanine lyase mutant exhibits a peptide fragment with decreased peak areas as shown in Table 4.

According to an embodiment of the present disclosure, the peptide map of the polyethylene glycol-modified phenylalanine lyase mutant is shown in FIG. 3.

In yet another aspect of the present disclosure, a method for preparing a polyethylene glycol-modified phenylalanine lyase mutant is provided, having one or more of the following features:

(1) The molar ratio of polyethylene glycol to phenylalanine lyase mutant in the modification reaction is (5 to 20):1 (polyethylene glycol to phenylalanine lyase mutant), preferably (8 to 16):1.

(2) The conjugation reaction system is a phosphate buffer with a pH range of 7.5 to 10.5.

The above method for preparing the polyethylene glycol-modified phenylalanine lyase mutant employs various purification methods to obtain a high-purity polyethylene glycol-modified phenylalanine lyase mutant.

In another preferred embodiment, purification of the modified sample is performed using techniques including, but not limited to, size-exclusion chromatography, ion exchange chromatography, hydrophobic interaction chromatography, tangential flow ultrafiltration, or a combination thereof. More preferably, size-exclusion chromatography and tangential flow ultrafiltration are used.

According to an embodiment of the present disclosure, the molar ratio of the PEG to the phenylalanine lyase mutant is (5 to 20):1, such as 5:1, 6:1, 7:1, 8:1, 9:1, 10:1, 11:1, 12:1, 13:1, 14:1, 15:1, 16:1, 17:1, 18:1, 19:1, and 20:1, and ranges between any two molar ratios, such as 8:1 to 20:1.

## Pharmaceutical composition

In yet another aspect of the present disclosure, a pharmaceutical composition is provided. According to an embodiment of the present disclosure, the pharmaceutical composition includes: the phenylalanine lyase mutant described above, or the phenylalanine lyase mutant conjugate or the pharmaceutically acceptable salt thereof described above. The pharmaceutical composition of the present disclosure is effective for preventing and/or treating hyperphenylalaninemia-related diseases, particularly the treatment of phenylketonuria.

According to an embodiment of the present disclosure, the pharmaceutical composition further includes a pharmaceutically acceptable carrier.

In still another aspect of the present disclosure, there is provided use of the phenylalanine lyase mutant described above, the phenylalanine lyase mutant conjugate or the pharmaceutically acceptable salt thereof described above, or the pharmaceutical composition described above in the manufacture of a medicament for preventing and/or treating a hyperphenylalaninemia-related disease.

In still another aspect of the present disclosure, there is provided use of the phenylalanine lyase mutant described above, the phenylalanine lyase mutant conjugate or the pharmaceutically acceptable salt thereof described above, or the pharmaceutical composition described above in the prevention and/or treatment of a hyperphenylalaninemia-related disease.

In still another aspect of the present disclosure, there is provided the phenylalanine lyase mutant described above, the phenylalanine lyase mutant conjugate or the pharmaceutically acceptable salt thereof described above, or the pharmaceutical composition described above for use in preventing and/or treating a hyperphenylalaninemia-related disease.

According to an embodiment of the present disclosure, each of the above uses may further include at least one of the following technical features:

According to an embodiment of the present disclosure, the hyperphenylalaninemia-related disease includes at least one of a tumor or cancer, phenylketonuria, and hyperphenylalaninemia.

According to an embodiment of the present disclosure, the tumor or cancer includes at least one of lung cancer, brain cancer, central nervous system cancer, colon cancer, prostate cancer, kidney cancer, head and neck cancer, ovarian cancer, uterine cancer, bladder cancer, breast cancer, pancreatic cancer, acute myeloid leukemia, acute lymphoblastic leukemia, myeloma, pediatric cancer, and resistant cancer.

**Method**

In still another aspect of the present disclosure, a method for preventing and/or treating hyperphenylalaninemia-related disease is provided. According to an embodiment of the present disclosure, the method includes: administering to a subject a pharmaceutically acceptable dose of the phenylalanine lyase mutant described above, the phenylalanine lyase mutant conjugate or the pharmaceutically acceptable salt thereof described above, or the pharmaceutical composition described above.

The effective amount of the phenylalanine lyase mutant, the phenylalanine lyase mutant conjugate or a pharmaceutically acceptable salt thereof, or the pharmaceutical composition according to the present disclosure may vary depending on the mode of administration, the severity of the disease to be treated, and other factors. Selection of a preferred effective amount can be determined by one of ordinary skill in the art based on various factors (e.g., through clinical trials). Such factors include, but are not limited to: the age, body weight, general health, sex, and diet of the subject, time of administration, drug combination, severity of the disease to be treated, route of administration, and the like. For example, divided doses may be administered several times per day as needed for treatment, e.g., four times per day, three times per day, twice per day, once per day, or once every other day; alternatively, the number of daily doses may be proportionally reduced; alternatively, the phenylalanine lyase mutant, the phenylalanine lyase mutant conjugate or the pharmaceutically acceptable salt thereof, or the pharmaceutical composition of the present disclosure is administered for at least one year or longer, preferably at least one month, more preferably at least one week, and most preferably at least one day, in order to achieve continuous remission of the tumor or cancer.

The phenylalanine lyase mutant, the phenylalanine lyase mutant conjugate or the pharmaceutically acceptable salt thereof, or the pharmaceutical composition of the present disclosure can be incorporated into suitable drugs which can be prepared in various forms such as liquid, semisolid, and solid dose forms and the like, including, but not limited to, solid dose forms, semisolid dose forms, liquid dose forms, and gaseous dose forms, and the like. A typical drug is in the form of a solution-type formulation, which can be administered by injection.

According to an embodiment of the present disclosure, the hyperphenylalaninemia-related disease includes at least one of a tumor or cancer, phenylketonuria, and hyperphenylalaninemia.

According to an embodiment of the present disclosure, the tumor or cancer includes at least one of lung cancer, brain cancer, central nervous system cancer, colon cancer, prostate cancer, kidney cancer, head and neck cancer, ovarian cancer, uterine cancer, bladder cancer, breast cancer, pancreatic cancer, acute myeloid leukemia, acute lymphoblastic leukemia, myeloma, pediatric cancer, and resistant cancer.

In yet another aspect, the present disclosure provides a method of reducing immunogenicity while maintaining enzymatic activity of phenylalanine lyase. According to an embodiment of the present disclosure, the method includes: performing PEG modification at a mutation site of a mutated phenylalanine lyase. The mutation site includes at least one of E72K, Q240K, R467K, and R544K. The method of the present disclosure reduces the immunogenicity of phenylalanine lyase while maintaining a high enzyme activity retention rate.

According to an embodiment of the present disclosure, the mutation site is defined with reference to an amino acid sequence as set forth in SEQ ID NO: 1.

According to an embodiment of the present disclosure, the mutated phenylalanine lyase is the phenylalanine lyase mutant as described above.

According to an embodiment of the present disclosure, the mutated phenylalanine lyase has an amino acid sequence as set forth in any one of SEQ ID NO: 4 to SEQ ID NO: 11, or an amino acid sequence having at least 50% homology thereto.

According to an embodiment of the present disclosure, the mutated phenylalanine lyase has a PEG modification at at least one of the amino acid positions $K^{72}$, $K^{240}$, $K^{467}$, or $K^{544}$. preferably, the mutated phenylalanine lyase has PEG modification at amino acid positions $K^{72}$, $K^{240}$, $K^{467}$, and $K^{544}$.

According to an embodiment of the present disclosure, polyethylene glycol used for the PEG modification has a molecular weight between 2 and 40 KDa, for example, between 2 and 10 KDa or between 5 and 20 KDa.

According to an embodiment of the present disclosure, the polyethylene glycol is a linear polyethylene glycol or a branched polyethylene glycol.

According to an embodiment of the present disclosure, the polyethylene glycol has a monomethoxy group or a hydroxyl group.

According to an embodiment of the present disclosure, the polyethylene glycol is a polyethylene glycol having a reactive group.

According to an embodiment of the present disclosure, the polyethylene glycol is conjugated to a lysine residue of the phenylalanine lyase mutant via an amide bond.

According to an embodiment of the present disclosure, the reactive group of the polyethylene glycol includes at least one of succinimidyl butyrate, succinimidyl propionate, succinimidyl acetate, succinimidyl carbonate, and nitrophenyl carbonate, preferably succinimidyl propionate.

The embodiments of the present disclosure will be explained with reference to the following examples. It will be understood by those skilled in the art that the following embodiments are illustrative only and are not to be construed as limiting the scope of the present disclosure. Where specific techniques or conditions are not specified in the embodiments, they are carried out according to techniques or conditions described in the literature in the art or according to the product specification. The reagents or instruments used, if not specified by the manufacturer, are conventional products available commercially.

Unless otherwise indicated, the practice of the present disclosure will employ conventional techniques in cell biology, molecular biology (including recombinant techniques), microbiology, biochemistry, and immunology, which are within the skill of the art. Such techniques are fully explained in the literature, such as Molecular Cloning: A Laboratory Manual, 2nd edition (Sambrook et al., 1989); Oligonucleotide Synthesis (ed. M.J. Gait, 1984), each of which is expressly incorporated herein by reference.

**Example 1: Prediction of phenylalanine lyase mutation sites**

The prediction of the mutation sites described in the present disclosure was performed through molecular modeling and simulation using online or offline software such as Verify_3D, ProSA-web, and SWISS-MODEL, combining results of T cell epitope/B cell epitope immunogenicity analysis to infer potential mutation regions, and the feasibility of PEGylated modification at the mutation sites was evaluated based on physicochemical properties after mutation. Taking phenylalanine lyase derived from *Anabaena variabilis* as an example, the physicochemical characteristics of lysine residues and mutated lysine sites were analyzed as follows:

Table 1-1: Analysis of physicochemical properties of lysine residues of phenylalanine lyase

| Residue No. | ASA | RSA | PK$_a$ | Location |
|---|---|---|---|---|
| K2 | | | | N-terminal residue |
| K10 | | | | N-terminal residue |
| K522 | 164.53 | 82.27% | 10.41/10.23 | |
| K195 | 138.10 | 69.05% | 10.38/10.31 | |
| K413 | 175.21 | 87.61% | 10.18/10.14 | |
| K493 | 122.45 | 61.23% | 9.48/9.77 | Adjacent residue |
| K494 | 110.90 | 55.45% | 9.87/9.91 | Adjacent residue |
| K145 | 73.46 | 36.73% | 11.25/11.08 | |
| K32 | 140.89 | 70.45% | 10.32/9.60 | Interfacing residue |
| K301 | 127.02 | 63.51% | 11.36/10.37 | Interfacing residue |
| K335 | 133.04 | 66.52% | 9.96/10.27 | |
| K115 | 82.60 | 41.30% | 8.03/8.83 | |
| K419 | 96.84 | 48.42% | 9.49/9.43 | |
| K109 | 54.97 | 27.49% | 10.33/6.25 | |
| K189 | 77.30 | 38.65% | 10.23/10.29 | |
| K216 | 0 | 0 | 9.05/8.82 | |
| K272 | 99.98 | 49.99% | 9.14/9.20 | Interfacing residue |
| K384 | 103.77 | 51.89% | 6.60/5.87 | Interfacing residue |

Table 1-2: Analysis of physicochemical characteristics of mutated lysine sites in phenylalanine lyase

| Residue No. | ASA | RSA | PK$_a$ | Location |
|---|---|---|---|---|
| R544K | 78.71 | 39.36% | 11.22/10.38 | Potential mutant site |
| R466K | 38.36 | 19.18% | 11.94/12.01 | Non-modifiable site |
| R467K | 84.84 | 42.42% | 14.02/13.51 | Potential mutant site |

(continued)

| Residue No. | ASA | RSA | PK$_a$ | Location |
|---|---|---|---|---|
| E72K | 128.74 | 64.37% | 10.32/10.35 | Potential mutant site |
| E75K | 84.13 | 42.07% | 10.61/10.57 | Interfacing residue |
| H240K | 62.26 | 31.13% | 10.78/9.68 | Potential mutant site |
| H250K | 0.26 | 0.13% | 6.94/7.24 | Non-modifiable site |

The physicochemical properties of lysine residues in the phenylalanine lyase sequence are shown in Table 1-1. Among them, K2 and K10 are N-terminal residues, which are theoretically modifiable sites; Based on physicochemical properties, K522, K195, K413, and K493/K494 are inferred to be modifiable lysine residues; K145, K32, K301, K335, K115, and K419 are inferred to be partially modified sites due to low solvent accessibility/PKa parameters or their location at the tetramer polymerization interface (interfacial amino acids); K109, K189, K216, K272, and K384 are inferred to have low probability or cannot be modified due to low solvent accessibility/Pka parameters and their location at the tetramer polymerization interface (interfacial amino acids).

Combined with the predicted immunogenic regions, the physicochemical characteristics of the proposed mutated lysine sites are shown in Table 1-2. Among them, R466K, E75K, and H250K are inferred to be partially modifiable or non-modifiable due to low solvent accessibility/Pka parameters or their location at the tetramer polymerization interface (interfacial amino acids). Therefore, R544K, R467K, E72K, and H240K were selected as candidate mutation sites.

**Example 2: Recombinant expression and preparation of phenylalanine lyase and its mutants**

The phenylalanine lyase and its mutants of the present disclosure were obtained using conventional methods in the art, with the specific steps as follows:

Full gene synthesis was performed to obtain the nucleotide sequences encoding the amino acid sequences of phenylalanine lyase or mutants thereof as set forth in SEQ ID NO: 1, SEQ ID NO: 4, SEQ ID NO: 5, and SEQ ID NO: 6. Expression plasmids pET-30a-PAL, pET-30a-PAL2, pET-30a-PAL3, and pET-30a-PAL4 were constructed by restriction endonuclease digestion and ligation with T4 DNA ligase using conventional methods in the art. The above four expression plasmids were transformed into E. coli, respectively, and the recombinant expression engineering bacteria were obtained by resistance screening. Then, YT culture medium was used for fermentation and culture, followed by IPTG induction, and the bacteria were collected by centrifugation and cryopreserved for future use.

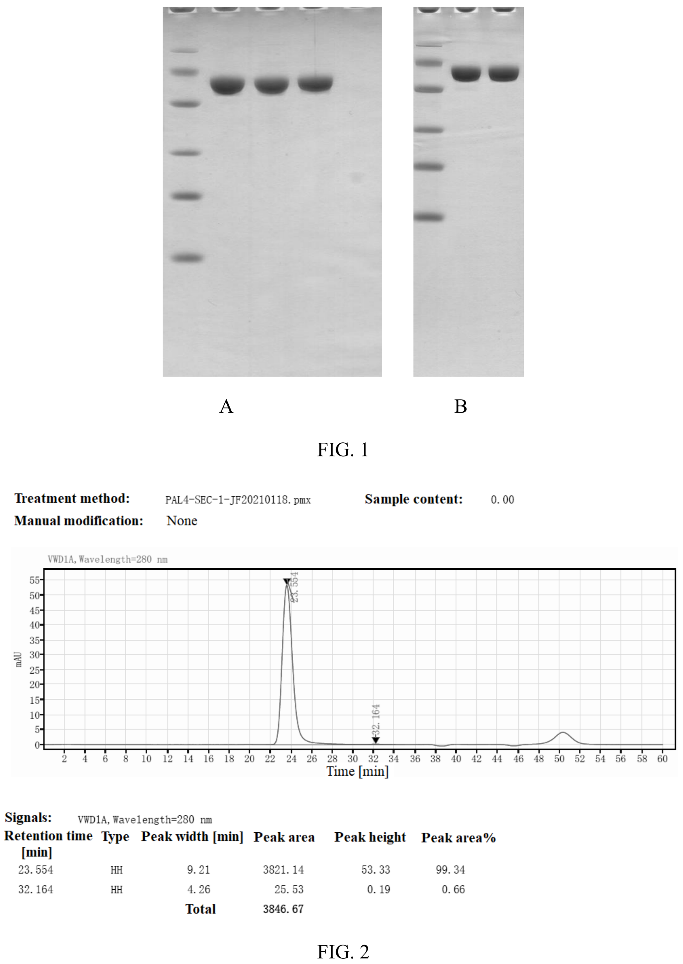

After physical or enzymatic lysis (using Tris-HCl buffer or $Na_2HPO_4$-$NaH_2PO_4$ buffer, commonly used in enzyme purification processes, to adjust pH to 8.0 $\pm$ 1.0), phenylalanine lyase or its mutant was distributed in soluble form in the supernatant of the lysate, and the insoluble lysis fragments were removed by centrifugation. $(NH_4)_2SO_4$ was added into the supernatant of the lysate after centrifugation to enrich phenylalanine lyase and reduce host protein impurities. The precipitate of phenylalanine lyase or its mutant was obtained by centrifugation again. After dissolution of the precipitate, host protein impurities were purified by hydrophobic chromatography, and the eluate was further purified by ion exchange chromatography to remove high- and low-molecular-weight related proteins. Hydrophobic chromatography media used were Phenyl or Butyl Sepharose, and ion exchange chromatography media used were Source 30Q or 15Q, yielding phenylalanine lyase and its mutants, which were named PAL protein, PAL2 protein, PAL3 protein, and PAL4 protein according to the number of point mutations. The purity of the intermediate product was then evaluated by SDS-PAGE and SEC-HPLC to be not less than 95%, and the high-molecular-weight polymer content was not more than 1.0%. The SDS-PAGE and SEC-HPLC detection profiles of the phenylalanine lyase mutant PAL4 protein are shown in FIG. 1A, FIG. 1B, and FIG. 2. In FIG. 1A, from left to right: middle-molecular-weight Marker (from top to bottom: 97.4 kDa, 66.2 kDa, 43.0 kDa, 31.0 kDa, 22.0 kDa, 14.4 kDa), PAL2, PAL3, PAL4; in FIG. 1B, from left to right: middle-molecular-weight Marker (from top to bottom: 97.4 kDa, 66.2 kDa, 43.0 kDa, 31.0 kDa, 22.0 kDa, 14.4 kDa), PAL, PAL4. FIG. 2 shows the SEC-HPLC profile of high- and low-molecular-weight content of the PAL4 mutant.

**Example 2: Preparation of polyethylene glycol conjugates of phenylalanine lyase and its mutants as well as their average degree of modification**

1. The PAL, PAL2, PAL3, and PAL4 proteins (phenylalanine lyase and its mutants thereof) prepared in Example 1 were subjected to ultrafiltration concentration and buffer exchange. In a 100 mmol/L $Na_2HPO_4$-$NaH_2PO_4$ buffer at pH 7.5-10.5, the concentration of phenylalanine lyase and its mutants was 20 mg/L. PEG-SPA-5K was added at different mass or molar ratios (see Table 1 for details), and the modification reaction was carried out at room temperature with stirring for 1 h. The resulting modified phenylalanine lyase and its mutant products were purified by ultrafiltration or size exclusion to remove free PEG and NHS, and the PEG-modified phenylalanine lyase and its mutant products were concentrated to above 5 mg/ml. Protein concentration was determined by the Lowry method, followed by sterile filtration to obtain polyethylene glycol conjugates (referred to as conjugates) of phenylalanine lyase and its mutants. The conjugates were named PAL, PAL2, PAL3, and PAL4 based on their mutation sites, and further designated according to the number of mutation sites and the molecular weight of PEG at different feed ratios, as specified in Table 1.

2. Determination of the average degree of modification of PEGylated conjugates of phenylalanine lyase and its mutants

Based on the linear relationship between the concentration of protein (phenylalanine lyase or its mutant) in solution and its refractive index (RI) and ultraviolet (UV) absorption, and the fact that polyethylene glycol had no UV absorption but exhibited a linear relationship between its concentration and refractive index (RI) absorption, and that the refractive index and ultraviolet absorptions of protein and PEG did not interfere with each other, a combination of SEC-HPLC with refractive index and ultraviolet detector could be used to establish calibration standard curves of protein and polyethylene glycol content versus peak area. The protein and polyethylene glycol contents in the test sample were calculated using these curves. Combined with the relative molecular weights of protein and polyethylene glycol, the molar ratio of protein to polyethylene glycol molecules was calculated and used as an indicator of the average degree of modification.

The calculation formula is as follows: [00174]

$$\frac{M\;(PAL)}{M\;(PEG)}\times\frac{\left[\dfrac{PL\;(RI)\times PAL\;(UV)}{PL\;(UV)}-PAL\;(RI)\right]}{1\mathrm{mg/ml\,PEG}\;(RI)},$$

where M represents relative molecular mass, PL represents PEGylated phenylalanine lyase, PAL represents phenylalanine lyase or its mutant, PEG represents polyethylene glycol, (RI) represents the refractive index peak area, and (UV) represents the ultraviolet peak area.

Table 1: Average degree of modification of phenylalanine lyase or its mutants at different feed ratios

| Name | No. | Molar ratio | Mass ratio | Average degree of modification |
|------|-----|-------------|------------|-------------------------------|
| PAL | PL5-1 | 1:6 | 1:8.7 | 8.20 |
| | PL5-2 | 1:8 | 1:11.6 | 9.04 |
| | PL5-3 | 1:12 | 1:17.4 | 10.28 |
| | PL5-4 | 1:14 | 1:20.3 | 10.59 |
| | PL5-5 | 1:16 | 1:23.2 | 10.73 |
| PAL2 | PL52-1 | 1:8 | 1:11.6 | 10.93 |
| | PL52-2 | 1:12 | 1:17.4 | 12.27 |
| | PL52-3 | 1:14 | 1:20.3 | 12.62 |
| | PL52-4 | 1:16 | 1:23.2 | 12.61 |
| PAL3 | PL53-1 | 1:8 | 1:11.6 | 10.70 |
| | PL53-2 | 1:12 | 1:17.4 | 11.90 |
| | PL53-3 | 1:14 | 1:20.3 | 12.20 |
| | PL53-4 | 1:16 | 1:23.2 | 12.64 |

(continued)

| Name | No. | Molar ratio | Mass ratio | Average degree of modification |
|------|-----|-------------|-----------|-------------------------------|
| PAL4 | PL54-1 | 1:6 | 1:8.7 | 10.54 |
| | PL54-2 | 1:8 | 1:11.6 | 11.46 |
| | PL54-3 | 1:12 | 1:17.4 | 12.82 |
| | PL54-4 | 1:14 | 1:20.3 | 13.23 |
| | PL54-5 | 1:16 | 1:23.2 | 13.60 |

It could be seen from the above that, compared with PAL, the average degree of modification of PAL2, PAL3, and PAL4 was higher, and especially under the condition of equal feed ratio, the average degree of modification of PAL4 > the average degree of modification of PAL3 > the average degree of modification of PAL2 > the average degree of modification of PAL. Therefore, it could be concluded that the number of mutation sites can effectively improve the average degree of modification under equal feeding ratio, and under equal feeding ratio, the average degree of modification of different structures was correlated with the number of mutation sites.

**Example 3: Specific enzyme activity of phenylalanine lyase and its mutants as well as their polyethylene glycol conjugates**

Phenylalanine lyase specifically catalyzes the conversion of phenylalanine into trans-cinnamic acid with the release of ammonia. Trans-cinnamic acid exhibits maximum light absorption at a wavelength of 290 nm. Enzyme activity is defined as the amount of enzyme required to produce 1 μmol of trans-cinnamic acid per minute in buffer (100 mmol/L Tris-HCl, pH 8.5) at room temperature (25°C) as one unit of enzyme activity.

1. Specific enzyme activity of phenylalanine lyase mutants

The specific enzyme activity of phenylalanine lyase and its mutants (i.e., PAL, PAL2, PAL3, and PAL4 proteins) prepared in Example 1 was evaluated by taking an appropriate amount of test sample and diluting it to 0.4 mg/ml using assay buffer (100 mmol/L Tris-HCl, pH 8.0). 2.9 ml of substrate solution (100 mmol/L Tris-HCl, 45 mmol/L Phe, pH 8.0) was placed in a cuvette, followed by addition of 0.1 ml of diluted test solution and immediate mixing. The changes in absorbance per minute were recorded at a wavelength of 290 nm, and the specific enzyme activity was calculated according to the formula.

$$\text{Enzyme activity (U/mg)} = (A_S - A_O)VN \times 10^6 / \varepsilon LTV_1$$

Where $A_S$ and $A_O$ represent the absorbance of trans-cinnamic acid at the beginning and end of the reaction, respectively; V is the total volume (L) of the reaction system; L is the optical path length of the cuvette (cm); $\varepsilon$ is the molar extinction coefficient of trans-cinnamic acid (10238.1 L/(mol·cm)); T is the reaction time (min); N is the dilution factor of the test sample; $V_1$ is the volume of test sample (ml).

Specific results are shown in Table 2. The enzyme activity retention rate was calculated by comparing the specific enzyme activity of phenylalanine lyase mutants with the specific enzyme activity of the phenylalanine lyase.

Table 2: Specific enzyme activity of phenylalanine lyase mutants

| Sample Name | PAL protein | PAL2 protein | PAL3 protein | PAL4 protein |
|-------------|-------------|--------------|--------------|--------------|
| Specific enzyme activity | 1.60 U/mg | 1.57 U/mg | 1.49 U/mg | 1.53 U/mg |

As shown in Table 2, there was no significant difference in the specific enzyme activity among PAL, PAL2, PAL3, and PAL4 proteins. Compared with PAL protein, the enzyme activity retention rate of phenylalanine lyase mutants (PAL2, PAL3, or PAL4 proteins) was over 90%.

2. Specific enzyme activity of polyethylene glycol conjugates of phenylalanine lyase mutants

The specific enzyme activity of the polyethylene glycol conjugates of phenylalanine lyase and its mutants prepared in Step 1 of Example 2 was determined according to the method described in Step 1 of this example. The enzyme activity retention rate was calculated by comparing the specific enzyme activity of each conjugate with its specific enzyme activity prior to PEG modification (e.g., the enzyme activity retention rate of PL5-1 = specific enzyme activity of PL5-1 / specific enzyme activity of PAL protein). See Table 3 for details.

Table 3: Specific enzyme activity of PEGylated phenylalanine lyase mutants

| Sample Name | Average degree of modification | Specific enzyme activity | Enzyme activity retention rate |
|---|---|---|---|
| PL5-1 | 8.20 | 1.39 | 87% |
| PL5-5 | 10.53 | 0.75 | 47% |
| PL52-4 | 12.61 | 1.05 | 67% |
| PL53-4 | 12.64 | 1.10 | 74% |
| PL54-1 | 10.54 | 1.47 | 96% |
| PL54-5 | 13.60 | 1.27 | 83% |

As can be seen from Table 3, the enzyme activity retention rate of the polyethylene glycol conjugates of the phenylalanine lyase mutant of the present disclosure after targeted saturation modification was above 65%, and among them, the polyethylene glycol conjugates of the phenylalanine lyase mutants at four mutation sites achieved an enzyme activity retention rate of over 80%. Furthermore, it was found that although phenylalanine lyase (unmutated structure) could reach a similar degree of modification by excessive reagent loading, its activity retention dropped significantly to only 47% of the pre-modification level, thereby indicating that for achieving saturation in average degree of modification, the PEGylated phenylalanine lyase mutant exhibits an average degree of modification and enzyme activity retention rate unattainable by the unmutated structure.

### Example 4: Study on modification sites of polyethylene glycol conjugates of PEGylated phenylalanine lyase and its mutants

PEGylated phenylalanine lyase and its mutants are formed by covalent conjugation of PEG to the amino side chain of lysine residues or the amino group of the N-terminal amino acid in the amino acid sequence. Since trypsin and lysyl endopeptidase cannot recognize and cleave PEG-modified lysine residues, the modification sites can be inferred by comparing peptide fragments before and after modification. The inventors analyzed the modification sites of the PEGylated phenylalanine lyase mutant by LC-MS using Trypsin and Lys-C for dual digestion of PEGylated phenylalanine lyase and its mutant (PL54-5 prepared in step 1 of Example 2) before and after polyethylene glycol covalent conjugation. Details are provided in Table 4 and FIG. 3.

The results showed that after polyethylene glycol modification and conjugation of the PAL4 protein to obtain PL54-5, the cleaved peptide fragments corresponding to the mutated lysine sites or their adjacent peptide fragments either completely disappeared or showed reduced response, indicating that all four mutation sites ($K^{72}$, $K^{240}$, $K^{467}$, and $K^{544}$) were fully modified, or the modification ratio was higher than 90%.

Table 4: Comparison of cleaved peptide maps before and after PL54 modification

| Peptide fragment | Amino number | Sequence | Molecular weight | PAL4 | PL54 | Remarks |
|---|---|---|---|---|---|---|
| T1 | 2 | MK | 277.382 | 278.4 | N.D. (not detected) | PEG modification site N-terminal site |
| T2 | 10 | TLSQAQSK | 861.950 | 863.0 | N.D. | PEG modification site |
| T3 | 32 | TSSQQFSFTGNS SANVIIGNQK | 2315.481 | 2317.4 | N.D. | PEG modification site (~50%, I.R.) |
| T4 | 40 | LTINDVAR | 901.030 | 902.0 | N.D. | |
| T5 | 43 | VAR | 344.414 | 345.4 | 345.4. | |

(continued)

| Peptide fragment | Amino number | Sequence | Molecular weight | PAL4 | PL54 | Remarks |
|---|---|---|---|---|---|---|
| T6 | 72 | NGTLVSLTNNTDILQGIQASCDYINNAVK | 3080.416 | 3080.4 | N.D. | Mutation site modified (> 95%) |
| T7 | 94 | SGEPIYGVTSGFGGMANVAISR | 2170.425 | 2170.4 | N.D. | |
| T8 | 109 | EQASELQTNLVWFLK | 1806.048 | 1806.0 | 1806.0 | Not modified |
| T9 | 115 | TGAGNK | 546.581 | 546.6 | 546.6 | Partial modified (20%) |
| T10 | 122 | LPLADVR | 782.938 | 782.9 | 782.9 | |
| T11 | 128 | AAMLLR | 673.872 | 673.9 | 673.9 | |
| T12 | 134 | ANSHMR | 714.797 | 714.8 | 714.8 | |
| T13 | 140 | GASGIR | 559.623 | 559.6 | 559.6 | |
| T14 | 145 | LELIK | 614.783 | 614.8 | 614.8 | Partial modified (~60%) |
| T15 | 146 | R | 174.203 | N.D. | N.D. | |
| T16 | 189 | MEIFLNAGVTPYVYEFGSIGASGDLVPLSYITGSLIGLDPSFK | 4540.204 | 4540.2 | 4540.2 | Not modified |
| T17 | 195 | VDFNGK | 678.743 | 678.7 | N.D. | PEG modification site |
| T18 | 204 | EMDAPTALR | 1003.138 | 1003.1 | N.D. | |
| T19 | 216 | QLNLSPLTLLPK | 1336.638 | 1336.6 | N.D. | Not modified (ASA=0) |
| T20 | 240 | EGLAMMNGTSVMTGIAANCVYDTK | 2477.863 | 2477.8 | N.D. | Mutation site modified (> 90%) |
| T21 | 272 | ILTAIAMGVHALDIQALNGTNQSFHPFIHNSK | 3459.971 | 3460.1 | N.D. | Not modified (I.R.) |
| T22 | 295 | PHPGQLWAADQMISLLANSQLVR | 2545.942 | 2545.9 | 2545.9 | |
| T23 | 301 | DELDGK | 675.693 | 675.7 | 675.7 | PEG modification site (~50%) |
| T24 | 305 | HDYR | 589.608 | 590.6 | 589.6 | |
| T25 | 313 | DHELIQDR | 1025.086 | 1025.1 | N.D. | |
| T26 | 317 | YSLR | 537.616 | 537.6 | N.D. | |

(continued)

| Peptide fragment | Amino number | Sequence | Molecular weight | PAL4 | PL54 | Remarks |
|---|---|---|---|---|---|---|
| T27 | 335 | CLPQYLGPIVDGISQIAK | 1915.278 | 1915.3 | 1915.3 | PEG modification site (~30%) |
| T28 | 376 | QIEIEINSVTDNPLIDVDNQASYHGGNFLGQYVGMGMDHLR | 4562.022 | 4562.0 | N.D. | |
| T29 | 384 | YYIGLLAK | 940.150 | 940.2 | 940.2 | Not modified (I.R.) |
| T30 | 410 | HLDVQIALLASPEFSNGLPPSLLGNR | 2759.157 | 2759.2 | 2759.2 | |
| T31 | 412 | ER | 303.318 | 303.3 | 303.3 | |
| T32 | 413 | K | 146.189 | N.D. | N.D. | PEG modification site (> 80%) |
| T33 | 419 | VNMGLK | 660.830 | 660.8 | 660.8 | PEG modification site (~30%) |
| T34 | 442 | GLQICGNSIMPLLTFYGNSIADR | 2483.883 | 2483.9 | 2483.8 | |
| T35 | 466 | FPTHAEQFNQNINSQGYTSATLAR | 2695.888 | 2695.9 | N.D. | |
| T36 | 467 | K | 146.189 | N.D. | N.D. | Mutation site modified (97%) |
| T37 | 490 | SVDIFQNYVAIALMFGVQAVDLR | 2570.001 | 2571.0 | N.D. | |
| T38 | 493 | TYK | 410.470 | 410.5 | N.D. | PEG modification site (> 80%) |
| T39 | 494 | K | 146.189 | N.D. | N.D. | PEG modification site (> 90%) |
| T40 | 501 | TGHYDAR | 818.844 | 818.8 | N.D. | |
| T41 | 510 | ASLSPATER | 931.013 | 931.0 | 931.0 | |
| T42 | 516 | LYSAVR | 707.828 | 707.8 | 707.8 | |
| T43 | 522 | HVVGQK | 3247.531 | 3247.5 | N.D. | PEG modification site (100%) |
| T44 | 544 | PTSDRPYIWNDNEQGLDEHIAK | 2598.771 | 2598.1 | N.D. | Mutation site modified (> 90%) |
| T45 | 567 | ISADIAAGGVIVQAVQDILPSLH | 2287.641 | 2287.6 | N.D. | C-terminal |

**Example 5: Multiple pharmacokinetic/immunogenicity study of poly(ethylene glycol) conjugates of phenylalanine lyase or mutants thereof in SD rats**

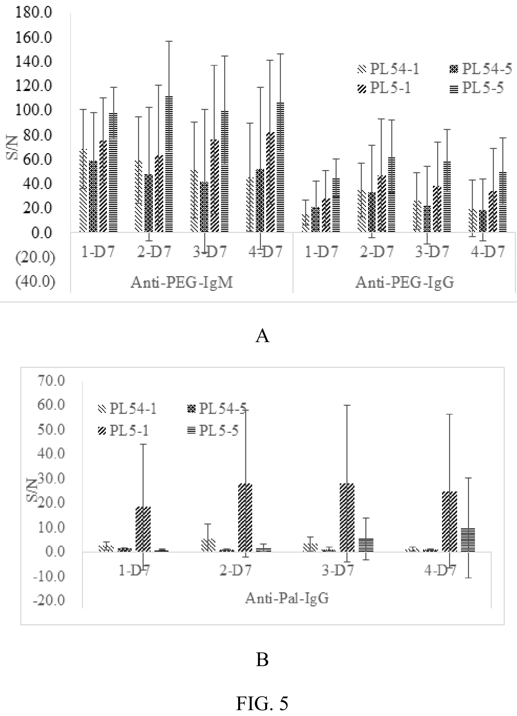

To evaluate PEGylated conjugates of phenylalanine lyase or its mutants with different average degrees of modification, the inventors divided SD rats into four groups (n=6 per group). The animals were subcutaneously injected with PL54-1 and PL54-5 (average degrees of modification: 10.54 and 13.60, respectively) and PL5-1 and PL5-5 (average degrees of modification: 8.2 and 10.53, respectively) from Table 3 of Example 2 at a dose of 2 mg/kg once a week for four doses. Blood samples were collected on D1 (Day 1), D3 (Day 3), and D7 (Day 7) after the first dose, as well as on D3 and D7 following each subsequent dose. The concentrations of PL54-1, PL54-5, PL5-1, and PL5-5 in rat serum were determined by sandwich ELISA to assess plasma drug concentration maintenance after multiple administrations. Anti-PEG-IgM/IgG antibody titers in rat serum from each group were measured using indirect ELISA (coating with PEG-BSA, adding positive rat anti-PEG Abs/test serum samples, followed by HRP-conjugated anti-rat IgM/IgG detection), while bridging ELISA (coating with PAL protein, adding positive sheep anti-PAL Abs/acidified serum samples, followed by biotin-labeled PAL detection) was also performed. The plasma drug concentration results are shown in FIG. 4, and the antibody detection results after administration were shown in FIG. 4 and FIG. 5.

As shown in FIG. 4, after the first administration, the serum concentrations of PEGylated conjugates of phenylalanine lyase or its mutants were approximately 30 to 40 $\mu$g/ml, with no significant difference. After the second administration, an accelerated clearance phenomenon was observed, and the plasma drug maintenance of PL54-5 was superior to that of PL5-1 and PL5-5. On Day 3 after the last administration, only 3/6 and 1/6 rats showed detectable plasma drug levels for PL5-1 and PL5-5, respectively, and plasma concentrations remained low in some animals. In contrast, 4/6 rats treated with PL54-5 (average degree of modification: 13.6) maintained high plasma drug concentrations ranging from 20 to 41 $\mu$g/ml. On Day 7 after the last administration, plasma concentrations of PL54-5 still maintained within the range of 3.2 to 3.9 $\mu$g/ml.

As shown in FIG. 5A, after multiple administrations, both anti-PEG-IgM and anti-PEG-IgG antibodies were positive in all four groups at a serum dilution of 1:20. Although differences in antibody intensity were observed, there was no clear correlation between antibody intensity and PEG dose (average degree of modification). The antibody responses induced by PL5-1/PL54-1 (low average modification degree, incomplete shielding) and PL5-5 (over-modified, reduced activity) were stronger than those induced by PL54-5.

As shown in FIG. 5B, at a serum dilution of 1:10, anti-PAL protein antibodies in PL54-1 and PL54-5 groups showed only very weak positivity after the first dose. The antibody titers generally decreased with four repeated dosing, and 7 days after the fourth dose, only 4/6 and 1/6 rats remained very weakly positive, respectively. For PL5-1, anti-PAL protein antibodies showed significant positivity after the first dose. With an increasing number of doses, the positive rate reached 6/6 7 days after the fourth dose, with 3/6 showing very significant positivity. The response pattern of PL5-5 was similar to that of PL5-1, with a positive rate of 5/6 at 7D after the fourth dose, of which 3/6 were significantly or very significantly positive. Furthermore, it can be observed that the accelerated clearance of plasma drug in rats is correlated with antibody intensity.

**Example 6: Pharmacodynamic and pharmacokinetic study in Pah[enu] mice after a single administration**

Phenylalanine hydroxylase (PAH) was central to phenylalanine metabolism in animals, catalyzing the conversion of phenylalanine to tyrosine. Deficiency of this gene underlies phenylketonuria. Missense mutations induced by ENU (Reference: Lukas Villiger et al., *Treatment of a metabolic liver disease by in vivo genome base editing in adult mice*; the missense mutation F263S) causes a severe decrease in PAH catalytic activity in homozygous mice, leading to accumulation of phenylalanine in serum that could not be metabolized, significantly higher than in normal wild-type mice. Therefore, the homozygous mouse carrying the missense mutation F263S can be used as an animal model for phenylketonuria (referred to as the Pah[enu] mouse or model mouse) in this drug study.

To evaluate the polyethylene glycol conjugate PL54 of the site-saturation modified phenylalanine lyase mutant, six Pah[enu] mice were assigned to three groups of two mice each. PL54-5 (average degree of modification 13.60, specific enzyme activity 1.27 U/mg) from Table 3 of Example 2 was administered to the phenylketonuria animal model mice at low, medium, and high doses of 0.5 mg/kg, 1.5 mg/kg, and 4.5 mg/kg, respectively, via subcutaneous injection. Blood samples were collected from mice in each group at 1 h before administration and at 1, 3, 5, 7, 10, and 14 days after administration, respectively, and the content of PL54-5 in serum was detected by sandwich ELISA (for details, see Example 5).

As shown in Table 5, when Pah[enu] mice were subcutaneously injected with PL54-5, the plasma drug concentration was directly proportional to the administration dose and maintained for 5 to 7 days.

Table 5: Blood drug concentration in serum after single administration in Pah<sup>enu</sup> mice

| Grouping | Animal # | D0 1:50 | 1-D1 1:500 | 1-D3 1:500 | 1-D5 1:500 | 1-D7 1:500 | 1-D10 1:500 | 1-D14 1:500 |
|---|---|---|---|---|---|---|---|---|
| 0.5 mg/kg s.c. | 8 | 0 | 0.3 | 2.4 | 0.9 | 0.2 | 0.1 | NF |
| 1.5mg/kg s.c. | 363 | 0 | 0.5 | 2.7 | 0.9 | 0.4 | 0 | 0 |
| | 381 | 0 | 0.6 | 2.2 | 0.9 | 0.3 | 0.1 | 0.1 |
| 4.5 mg/kg s.c. | 2 | 0 | 1.6 | 2.6 | 1.9 | 0.8 | 0 | NF |
| | 379 | 0 | 0.8 | 2.9 | 1.2 | 0.4 | NF | 0.2 |

**Example 7: Pharmacokinetics, pharmacodynamics, and immunogenicity study in Pah<sup>enu</sup> mice after multiple administrations**

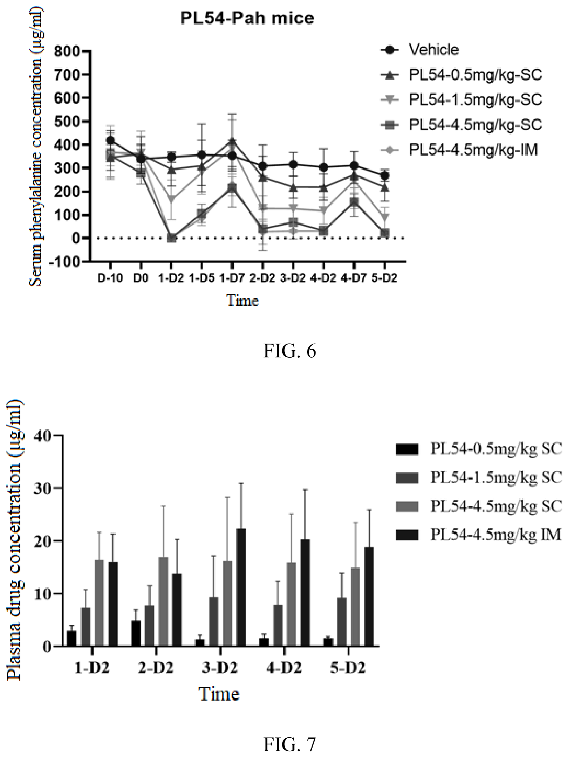

To evaluate the polyethylene glycol conjugate PL54 of site-saturation modified phenylalanine lyase mutant, 27 Pah<sup>enu</sup> mice were allocated into five groups (four experimental groups and one control group), with six mice in each group. PL54-5 (average degree of modification 13.60, specific enzyme activity 1.27 U/mg) from Table 3 of Example 2 was administered to phenylketonuria animal model mice at low, medium, and high doses of 0.5 mg/kg, 1.5 mg/kg, and 4.5 mg/kg, respectively, via subcutaneous injection. The high-dose group of 4.5 mg/kg also included a group receiving intramuscular administration, once per week. The control group was administered phosphate buffered saline as a placebo subcutaneously once weekly. Blood samples were collected from mice in each group at 1 h before administration and at 2, 5, and 7 days after administration for each group. The content of PL54-5 in serum, changes in phenylalanine concentration (Reference: Judy Peat et al., *Determination of Phenylalanine and Tyrosine by High Performance Liquid Chromatography-Tandem Mass Spectrometry),* and Anti-PEG-IgM/IgG antibody titers (see Example 5 for details) were detected by sandwich ELISA (see Example 5 for details), and their pharmacokinetics were evaluated, see FIG. 6 to FIG.9 for details.

(1) Pharmacodynamic study in Pah<sup>enu</sup> mice after multiple administrations

The changes in serum phenylalanine concentrations in Pah<sup>enu</sup> mice after multiple administrations at different doses are shown in FIG. 6. The results showed that subcutaneous administration at medium and high doses (1.5 mg/kg and 4.5 mg/kg) effectively reduced serum phenylalanine levels, with a reduction of approximately 30%-50% at the medium dose. At the high dose, serum phenylalanine could be reduced below the method detection limit within 2 days after the first administration, and the efficacy lasted for 5 to 7 days following the initial dose. After five repeated administrations, the maintenance of efficacy was similar to the reduction in serum phenylalanine levels following the first dose, with the medium-dose group showing a decrease of approximately 75% and the high-dose group exhibiting a reduction of more than 90% within 2 days. The efficacy after intramuscular administration was consistent with that after subcutaneous administration.

(2) Pharmacokinetics of serum in Pah<sup>enu</sup> mice after multiple administrations

The changes in plasma drug concentration in Pah<sup>enu</sup> mice after multiple administrations at different doses are shown in FIG. 7. The results showed that two days after subcutaneous administration, the plasma drug concentration was directly proportional to the administration dose. After continuous administration for five weeks, plasma drug concentration and serum phenylalanine reduction showed a clear dose-dependent relationship, demonstrating a good PK-PD correlation. The plasma drug concentration in the high-dose intramuscular administration group was higher than that in the subcutaneous administration group, suggesting that intramuscular administration may have higher bioavailability.

(3) Immunogenicity study in Pah<sup>enu</sup> mice after multiple administrations

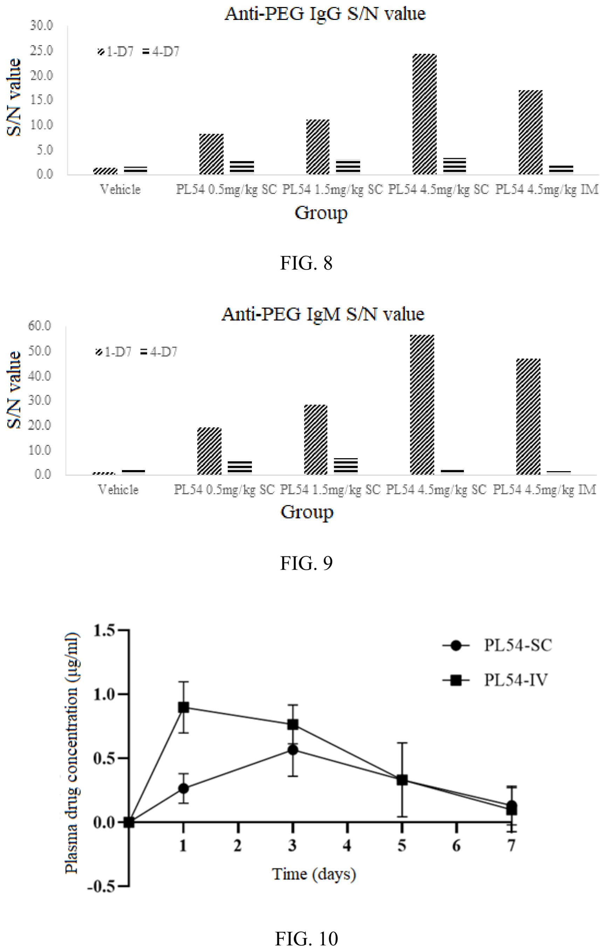

The anti-PEG and anti-PAL antibody titers in Pah<sup>enu</sup> mice following multiple administrations at different doses are shown in FIG. 8 and FIG. 9. The results showed that anti-PEG antibodies were positive in all dose groups after administration, and the antibody titer was positively correlated with the administration dose. S/N values of IgG and IgM antibodies decreased with increasing number of administrations, and the decrease was more pronounced in the high-dose group. The anti-PAL4 protein antibodies were negative in the subcutaneous administration group, while weak positivity was observed in the intramuscular administration group.

**Example 8: Comparative study on immunogenicity of polyethylene glycol conjugates with different molecular weights in rats**

Polyethylene glycol conjugates of phenylalanine lyase or its mutant with different structures and modifier molecular weights, namely PL20, PL24, and PL54, were prepared according to the method described in Example 2. The phenylalanine lyase in PL20 has the amino acid sequence as set forth in SEQ ID NO: 1, and the polyethylene glycol has a molecular weight of 20K. The phenylalanine lyase in PL24 has the amino acid sequence as set forth in SEQ ID NO: 6, and the polyethylene glycol has a molecular weight of 2K. The phenylalanine lyase in PL54 has the amino acid sequence as set forth in SEQ ID NO: 6, and the polyethylene glycol has a molecular weight of 5K.

To evaluate the immunogenicity of PL20, PL24, and PL54 in animals, normal rats were selected and divided into 3 groups, with 6 rats per group (3 males and 3 females). Subcutaneous administration was performed at a dose of 0.75 mg/kg with PL54 (average degree of modification 13.6, 1.27 U/mg), PL24 (average degree of modification 13.3, 0.96 U/mg), and PL20 (average degree of modification 7.6, 1.14 U/mg) for the three groups, respectively. Administration was repeated 7 times, once weekly. Blood samples were collected 7 days after each administration. Serum anti-PEG-IgG and anti-PAL4 protein antibodies were detected by ELISA (refer to Example 5 for details). Results are shown in Table 6. In the table, "1-7" indicated 7 days after the first administration, and "7-7" indicated 7 days after the seventh administration. x represented the average OD value of antibody detection by ELISA, and the positive rate represented the ratio of the number of antibody-positive animals to the total number of animals in the group.

Table 6: Antibody titers in rats after multiple administrations of polyethylene glycol conjugates with different molecular weights

| Drug structure | Comparison of ADA test data 7 days after the first and seventh administration | | | |
| --- | --- | --- | --- | --- |
| | anti-PEG-IgG | | anti-PAL-IgG | |
| | 1-7 | 7-7 | 1-7 | 7-7 |
| PL20 (s.c.) | $\bar{x}$=0.398 Positive rate: 6/6 | $\bar{x}$=0.768 Positive rate: 6/6 | $\bar{x}$=0.118 Positive rate: 1/6 | $\bar{x}$=0.102 Positive rate: 1/6 |
| PL24 (s.c.) | $\bar{x}$=0.330 Positive rate: 6/6 | $\bar{x}$=0.368 Positive rate: 6/6 | $\bar{x}$=0.130 Positive rate: 4/6 | $\bar{x}$=0.891 Positive rate: 6/6 |
| PL54 (s.c.) | $\bar{x}$=0.384 Positive rate: 2/6 | $\bar{x}$=0.304 Positive rate: 6/6 | $\bar{x}$=0.107 Positive rate: 2/6 | $\bar{x}$=0.089 Positive rate: 0/6 |

As shown in Table 6, the anti-PEG-IgG antibody titer in rats 7 days after the seventh administration of PL20 was higher than that in the PL24 and PL54 groups. No anti-PAL4 protein antibody was detected in animals 7 days after seven doses of PL54, indicating superior performance compared to PL20 and PL24.

**Example 9: Subcutaneous/intravenous pharmacokinetics study in beagle dogs after multiple administrations**

Pharmacokinetics after multiple subcutaneous or intravenous administrations was evaluated in dogs. Six beagle dogs were equally divided into two groups: the subcutaneous administration group and the intravenous administration group. The drug used was PL54-5 (average degree of modification 13.60, specific enzyme activity 1.27 U/mg) from Table 3 of Example 2, administered at an administration dose of 2 mg/kg once weekly. Blood samples were collected from both groups on days 1, 3, 5, and 7 after the first administration, and on Day 1 and Day 3 after each subsequent administration. Serum plasma drug concentrations were measured using the double-antibody sandwich ELISA method (see Example 5 for details). The plasma drug concentration analysis results are shown in FIG. 10.

It can be seen from FIG. 10 that the peak time of blood drug concentration after single subcutaneous or intravenous administration was 3 days and 1 day after administration, respectively. The bioavailability of subcutaneous administration relative to intravenous administration was 64.4%. After multiple administrations, accelerated blood clearance was observed in 1/3 of dogs following subcutaneous administration and in 2/3 of dogs following intravenous administration.

**Example 10: Comparative study on pharmacodynamics and immunogenicity of polyethylene glycol conjugate PL54-5 and Pegvaliase in Pah^enu mice after multiple administrations**

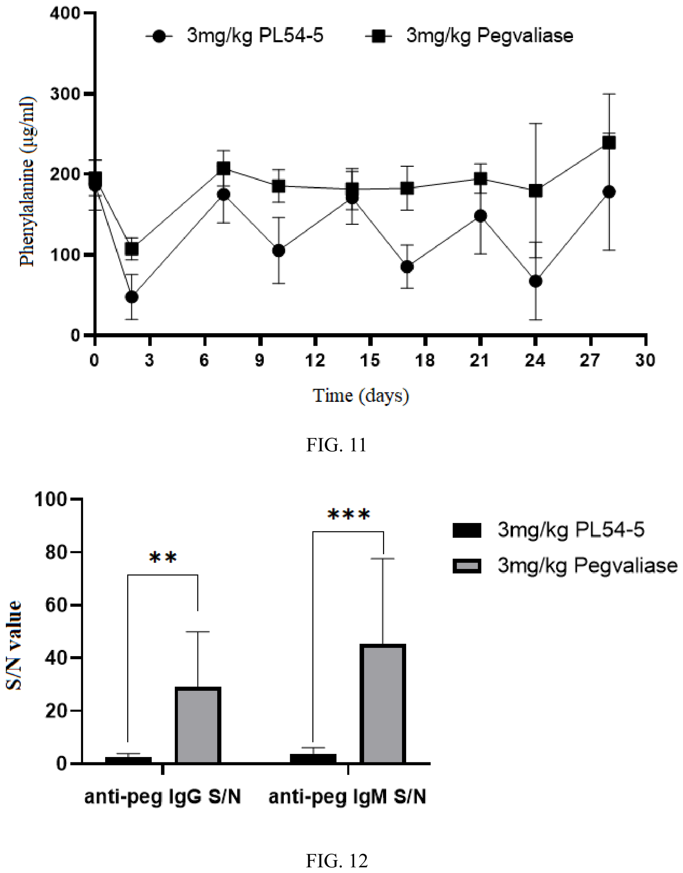

To evaluate the differences in pharmacodynamics and immunogenicity between the polyethylene glycol conjugate PL54-5 of directionally saturated modified phenylalanine lyase mutant and Pegvaliase (marketed drug), eight Pah$^{enu}$ mice were allocated into two groups of four mice each, and administered with either PL54-5 (average degree of modification: 13.60, specific enzyme activity: 1.27 U/mg) from Table 3 of Example 2 or Pegvaliase Injection (manufactured by BioMarin, purchased lot number L161239, specification: 20 mg/mL/vial) at an administration dose of 3.0 mg/kg, once weekly via subcutaneous injection for four consecutive weeks. Blood samples were collected from each group of mice before administration and at 3 days and 7 days after each administration, respectively. Changes in serum phenylalanine concentration were measured (Reference: Judy Peat et al., Determination of Phenylalanine and Tyrosine by High Performance Liquid Chromatography-Tandem Mass Spectrometry) and anti-PEG-IgM/IgG antibody titers of each animal were determined at the end of the study (see Example 5 for details), to assess differences in pharmacodynamics and immunogenicity, as shown in FIG. 11 and FIG. 12.

(1) Comparison of pharmacodynamic studies in Pah$^{enu}$ mice after multiple administrations

The changes in serum phenylalanine concentration in Pah$^{enu}$ mice after multiple administrations of PL54-5 and Pegvaliase are shown in FIG. 11. The results showed that at the same dose (3 mg/kg), both PL54-5 and Pegvaliase exhibited similar phenylalanine-lowering effects on Day 3 of the first administration, with levels returning to baseline by Day 7. However, during the second to fourth administrations, PL54-5 consistently reduced phenylalanine levels on Day 3, maintaining efficacy similar to the first administration, whereas Pegvaliase lost its ability to reduce phenylalanine. Long-term administration of PL54-5 demonstrated significantly superior phenylalanine-lowering effects compared to Pegvaliase.

(2) Immunogenicity study in Pah$^{enu}$ mice after multiple administrations

The anti-PEG antibody titers in Pah$^{enu}$ mice after multiple administrations of PL54-5 and Pegvaliase are shown in FIG. 12. The results showed that after four weeks of continuous administration of 3 mg/kg PL54-5 and 3 mg/kg Pegvaliase, the S/N values of anti-PEG IgG and IgM antibodies in the Pegvaliase group were significantly higher than those in the PL54-5 group. PL54-5 exhibited significantly lower immunogenicity than pegvaliase after long-term administration in Pah$^{enu}$ mice.

In this specification, references to "one embodiment", "some embodiments", "an example", "a specific example", or "some examples" are intended to indicate that the particular features, structures, materials, or characteristics described in connection with the embodiment or example are included in at least one embodiment or example of the present disclosure. In this specification, schematic descriptions of the above terms do not necessarily refer to the same embodiment or example. Moreover, the specific features, structures, materials, or characteristics described may be combined in any suitable manner in one or more embodiments or examples. Furthermore, unless otherwise contradictory, those skilled in the art may combine and integrate different embodiments or examples and their features described in this specification.

Although embodiments of the present disclosure have been shown and described, it is understood that these embodiments are illustrative and not limiting, and that various changes, modifications, replacements, and alterations may be made to the above-described embodiments by those of ordinary skill in the art within the scope of the present disclosure.

**Claims**

1. A phenylalanine lyase mutant, wherein, compared with an amino acid sequence of phenylalanine lyase, the phenylalanine lyase mutant has a mutation site at least one of site 72, site 240, site 467, and site 544.

2. The phenylalanine lyase mutant according to claim 1, wherein the mutation site is defined with reference to an amino acid sequence as set forth in SEQ ID NO: 1.

3. The phenylalanine lyase mutant according to claim 1, wherein the phenylalanine lyase has an amino acid sequence as set forth in SEQ ID NO: 1 to SEQ ID NO: 3, or an amino acid sequence having at least 50% homology thereto.

4. The phenylalanine lyase mutant according to claim 1, wherein the mutation site of the phenylalanine lyase mutant corresponds to an amino acid capable of conjugating with a modifying group.

5. The phenylalanine lyase mutant according to claim 4, wherein the amino acid capable of conjugating with a modifying group is lysine.

6. The phenylalanine lyase mutant according to claim 1, wherein the mutation site of the phenylalanine lyase mutant corresponds to an amino acid having an accessible surface area, ASA, value of ≥ 60% or a relative solvent accessibility, RSA, value of ≥ 30%.

7. The phenylalanine lyase mutant according to claim 1, wherein the mutation site of the phenylalanine lyase mutant corresponds to an amino acid having a pKa value > 9.0.

8. The phenylalanine lyase mutant according to claim 1, wherein, compared with an amino acid sequence as set forth in SEQ ID NO: 1 or SEQ ID NO: 2, the phenylalanine lyase mutant comprises at least one mutation site of E72K, Q240K, R467K, and R544K.

9. The phenylalanine lyase mutant according to claim 1, wherein, compared with an amino acid sequence as set forth in SEQ ID NO: 3, the phenylalanine lyase mutant comprises at least one mutation site of E72K, R467K, and R544K.

10. The phenylalanine lyase mutant according to claim 1, wherein, compared with an amino acid sequence as set forth in SEQ ID NO: 1 or SEQ ID NO: 2, the phenylalanine lyase mutant comprises:

    1) mutation sites of R467K and R544K;
    2) mutation sites of E72K, R467K, and R544K; or
    3) mutation sites of E72K, Q240K, R467K, and R544K.

11. The phenylalanine lyase mutant according to claim 1, wherein, compared with an amino acid sequence as set forth in SEQ ID NO: 3, the phenylalanine lyase mutant comprises:

    1) mutation sites of R467K and R544K; or
    2) mutation sites of E72K, R467K, and R544K.

12. The phenylalanine lyase mutant according to claim 1, wherein the phenylalanine lyase mutant has an amino acid sequence as set forth in any one of SEQ ID NO: 4 to SEQ ID NO: 11, or an amino acid sequence having at least 50% homology thereto.

13. A nucleic acid molecule, encoding the phenylalanine lyase mutant according to any one of claims 1 to 12, optionally, the nucleic acid molecule is DNA.

14. An expression vector, carrying the nucleic acid molecule according to claim 13, optionally, the expression vector is a eukaryotic expression vector, a prokaryotic expression vector, a virus, or a bacteriophage, and preferably, the expression vector is a lentiviral expression vector.

15. A recombinant cell, carrying the nucleic acid molecule according to claim 13 or the expression vector according to claim 14, or expressing the phenylalanine lyase mutant according to any one of claims 1 to 12, optionally, the recombinant cell is obtained by introducing the expression vector according to claim 14 into a host cell.

16. A phenylalanine lyase mutant conjugate or a pharmaceutically acceptable salt thereof, comprising:

    the phenylalanine lyase mutant according to any one of claims 1 to 12; and
    a modifying group conjugated to the phenylalanine lyase mutant.

17. The phenylalanine lyase mutant conjugate or the pharmaceutically acceptable salt thereof according to claim 16, wherein the modifying group is polyethylene glycol.

18. The phenylalanine lyase mutant conjugate or the pharmaceutically acceptable salt thereof according to claim 17, wherein the polyethylene glycol has a molecular weight of 2 to 40 KDa.

19. The phenylalanine lyase mutant conjugate or the pharmaceutically acceptable salt thereof according to claim 17, wherein the polyethylene glycol has a molecular weight of 2 to 10 KDa.

20. The phenylalanine lyase mutant conjugate or the pharmaceutically acceptable salt thereof according to claim 17, wherein the polyethylene glycol is linear polyethylene glycol or branched polyethylene glycol.

21. The phenylalanine lyase mutant conjugate or the pharmaceutically acceptable salt thereof according to claim 17, wherein the polyethylene glycol has a monomethoxy group or a hydroxyl group.

22. The phenylalanine lyase mutant conjugate or the pharmaceutically acceptable salt thereof according to claim 17, wherein the polyethylene glycol is polyethylene glycol having an active group.

23. The phenylalanine lyase mutant conjugate or the pharmaceutically acceptable salt thereof according to claim 17, wherein the active group of the polyethylene glycol comprises at least one of succinimidyl butyrate, succinimidyl propionate, succinimidyl acetate, succinimidyl carbonate, and nitrophenyl carbonate.

24. The phenylalanine lyase mutant conjugate or the pharmaceutically acceptable salt thereof according to claim 17, wherein the active group of the polyethylene glycol is succinimidyl propionate.

25. The phenylalanine lyase mutant conjugate or the pharmaceutically acceptable salt thereof according to claim 16, wherein the modifying group is conjugated to a lysine residue of the phenylalanine lyase mutant via an amide bond.

26. The phenylalanine lyase mutant conjugate or the pharmaceutically acceptable salt thereof according to claim 16, wherein a conjugation proportion of lysine modified with the modifying group in the phenylalanine lyase mutant conjugate or the pharmaceutically acceptable salt thereof is not less than 50%.

27. The phenylalanine lyase mutant conjugate or the pharmaceutically acceptable salt thereof according to claim 16, wherein a conjugation proportion of lysine modified with the modifying group at the mutation site in the phenylalanine lyase mutant conjugate or the pharmaceutically acceptable salt thereof is not less than 80%.

28. The phenylalanine lyase mutant conjugate or the pharmaceutically acceptable salt thereof according to claim 16, wherein a molar ratio of the modifying group to the phenylalanine lyase mutant is (5 to 20):1.

29. The phenylalanine lyase mutant conjugate or the pharmaceutically acceptable salt thereof according to claim 16, wherein a peptide map of the phenylalanine lyase mutant conjugate exhibits a peptide fragment with decreased peak area as shown in Table 4.

30. The phenylalanine lyase mutant conjugate or the pharmaceutically acceptable salt thereof according to claim 16, wherein a peptide map of the phenylalanine lyase mutant conjugate is as shown in FIG. 3.

31. A polyethylene glycol-modified phenylalanine lyase mutant, having PEG modification at at least the following amino acid positions:
$K^{72}$, $K^{240}$, $K^{467}$, and $K^{544}$.

32. The polyethylene glycol-modified phenylalanine lyase mutant according to claim 31, wherein polyethylene glycol used for the PEG modification has a molecular weight of 2 to 40 KDa.

33. The polyethylene glycol-modified phenylalanine lyase mutant according to claim 31, wherein polyethylene glycol used for the PEG modification has a molecular weight of 2 to 10 KDa.

34. The polyethylene glycol-modified phenylalanine lyase mutant according to claim 32 or 33, wherein the polyethylene glycol is linear polyethylene glycol or branched polyethylene glycol.

35. The polyethylene glycol-modified phenylalanine lyase mutant according to claim 32 or 33, wherein the polyethylene glycol has a monomethoxy group or a hydroxyl group.

36. The polyethylene glycol-modified phenylalanine lyase mutant according to claim 32 or 33, wherein the polyethylene glycol is polyethylene glycol having an active group.

37. The polyethylene glycol-modified phenylalanine lyase mutant according to claim 32 or 33, wherein the polyethylene glycol is conjugated to a lysine residue of the phenylalanine lyase mutant via an amide bond.

38. The polyethylene glycol-modified phenylalanine lyase mutant according to claim 32 or 33, wherein the active group of polyethylene glycol comprises at least one of succinimidyl butyrate, succinimidyl propionate, succinimidyl acetate, succinimidyl carbonate, and nitrophenyl carbonate.

39. The polyethylene glycol-modified phenylalanine lyase mutant according to claim 32 or 33, wherein the active group of polyethylene glycol is succinimidyl propionate.

40. The polyethylene glycol-modified phenylalanine lyase mutant according to claim 31, wherein a molar ratio of PEG to the phenylalanine lyase mutant is (5 to 20):1.

41. The polyethylene glycol-modified phenylalanine lyase mutant according to claim 31, wherein the phenylalanine lyase mutant is the phenylalanine lyase mutant as defined in any one of claims 1 to 12.

42. The polyethylene glycol-modified phenylalanine lyase mutant according to claim 31, wherein the phenylalanine lyase mutant has an amino acid sequence as set forth in any one of SEQ ID NO: 4 to SEQ ID NO: 11, or an amino acid sequence having at least 50% homology thereto.

43. The polyethylene glycol-modified phenylalanine lyase mutant according to claim 31, wherein a peptide map of the polyethylene glycol-modified phenylalanine lyase mutant exhibits a peptide fragment with decreased peak area as shown in Table 4.

44. The polyethylene glycol-modified phenylalanine lyase mutant according to claim 31, wherein a peptide map of the polyethylene glycol-modified phenylalanine lyase mutant is as shown in FIG. 3.

45. A pharmaceutical composition, comprising:

the phenylalanine lyase mutant according to any one of claims 1 to 12, the phenylalanine lyase mutant conjugate or the pharmaceutically acceptable salt thereof according to any one of claims 16 to 30, or the polyethylene glycol-modified phenylalanine lyase mutant according to any one of claims 31 to 44; and
optionally, a pharmaceutically acceptable carrier.

46. Use of the phenylalanine lyase mutant according to any one of claims 1 to 12, the phenylalanine lyase mutant conjugate or the pharmaceutically acceptable salt thereof according to any one of claims 16 to 30, the polyethylene glycol-modified phenylalanine lyase mutant according to any one of claims 31 to 44, or the pharmaceutical composition according to claim 45 in the manufacture of a medicament for preventing and/or treating a hyperphenylalaninemia-related disease,

optionally, the hyperphenylalaninemia-related disease comprises at least one of a tumor or cancer, phenylketonuria, and hyperphenylalaninemia;
optionally, the tumor or cancer comprises at least one of lung cancer, brain cancer, central nervous system cancer, colon cancer, prostate cancer, kidney cancer, head and neck cancer, ovarian cancer, uterine cancer, bladder cancer, breast cancer, pancreatic cancer, acute myeloid leukemia, acute lymphoblastic leukemia, myeloma, pediatric cancer, and resistant cancer.

47. Use of the phenylalanine lyase mutant according to any one of claims 1 to 12, the phenylalanine lyase mutant conjugate or the pharmaceutically acceptable salt thereof according to any one of claims 16 to 30, the polyethylene glycol-modified phenylalanine lyase mutant according to any one of claims 31 to 44, or the pharmaceutical composition according to claim 45 in the prevention and/or treatment of a hyperphenylalaninemia-related disease,

optionally, the hyperphenylalaninemia-related disease comprises at least one of a tumor or cancer, phenylketonuria, and hyperphenylalaninemia;
optionally, the tumor or cancer comprises at least one of lung cancer, brain cancer, central nervous system cancer, colon cancer, prostate cancer, kidney cancer, head and neck cancer, ovarian cancer, uterine cancer, bladder cancer, breast cancer, pancreatic cancer, acute myeloid leukemia, acute lymphoblastic leukemia, myeloma, pediatric cancer, and resistant cancer.

48. The phenylalanine lyase mutant according to any one of claims 1 to 12, the phenylalanine lyase mutant conjugate or the pharmaceutically acceptable salt thereof according to any one of claims 16 to 30, the polyethylene glycol-modified phenylalanine lyase mutant according to any one of claims 31 to 44, or the pharmaceutical composition according to claim 45 for use in preventing and/or treating a hyperphenylalaninemia-related disease,

optionally, the hyperphenylalaninemia-related disease comprises at least one of a tumor or cancer, phenylketonuria, and hyperphenylalaninemia;

optionally, the tumor or cancer comprises at least one of lung cancer, brain cancer, central nervous system cancer, colon cancer, prostate cancer, kidney cancer, head and neck cancer, ovarian cancer, uterine cancer, bladder cancer, breast cancer, pancreatic cancer, acute myeloid leukemia, acute lymphoblastic leukemia, myeloma, pediatric cancer, and resistant cancer.

49. A method for preventing and/or treating hyperphenylalaninemia-related disease, comprising:

administering to a subject a pharmaceutically acceptable dose of the phenylalanine lyase mutant according to any one of claims 1 to 12, the phenylalanine lyase mutant conjugate or the pharmaceutically acceptable salt thereof according to any one of claims 16 to 30, the polyethylene glycol-modified phenylalanine lyase mutant according to any one of claims 31 to 44, or the pharmaceutical composition according to claim 45,

optionally, the hyperphenylalaninemia-related disease comprises at least one of a tumor or cancer, phenylketonuria, and hyperphenylalaninemia;

optionally, the tumor or cancer comprises at least one of lung cancer, brain cancer, central nervous system cancer, colon cancer, prostate cancer, kidney cancer, head and neck cancer, ovarian cancer, uterine cancer, bladder cancer, breast cancer, pancreatic cancer, acute myeloid leukemia, acute lymphoblastic leukemia, myeloma, pediatric cancer, and resistant cancer.

50. A method for reducing immunogenicity while maintaining enzymatic activity of phenylalanine lyase, comprising:

performing PEG modification at a mutation site of a mutated phenylalanine lyase,
wherein the mutation site comprises at least one of E72K, Q240K, R467K, and R544K.

51. The method according to claim 50, wherein the mutation site is defined with reference to an amino acid sequence as set forth in SEQ ID NO: 1.

52. The method according to claim 50, wherein the mutated phenylalanine lyase is the phenylalanine lyase mutant according to any one of claims 1 to 12.

53. The method according to claim 50, wherein the mutated phenylalanine lyase has an amino acid sequence as set forth in any one of SEQ ID NO: 4 to SEQ ID NO: 11, or an amino acid sequence having at least 50% homology thereto.

54. The method according to claim 50, wherein the mutated phenylalanine lyase has PEG modification at amino acid positions $K^{72}$, $K^{240}$, $K^{467}$, and $K^{544}$.

55. The method according to claim 50, wherein polyethylene glycol used for the PEG modification has a molecular weight of from 2 to 40 KDa.

56. The method according to claim 50, wherein polyethylene glycol used for the PEG modification has a molecular weight of from 2 to 10 KDa.

57. The method according to claim 55 or 56, wherein the polyethylene glycol is linear polyethylene glycol or branched polyethylene glycol.

58. The method according to claim 55 or 56, wherein the polyethylene glycol has a monomethoxy group or a hydroxyl group.

59. The method according to claim 55 or 56, wherein the polyethylene glycol is a polyethylene glycol having a reactive group.

60. The method according to claim 55 or 56, wherein the polyethylene glycol is conjugated to a lysine residue of the phenylalanine lyase mutant via an amide bond.

61. The method according to claim 55 or 56, wherein the reactive group of the polyethylene glycol comprises at least one of succinimidyl butyrate, succinimidyl propionate, succinimidyl acetate, succinimidyl carbonate, and nitrophenyl carbonate.

62. The method according to claim 55 or 56, wherein the reactive group of the polyethylene glycol is succinimidyl propionate.

A                                              B

FIG. 1

**Treatment method:**    PAL4-SEC-1-JF20210118.pmx        **Sample content:**    0.00

**Manual modification:**    None

**Signals:**    VWD1A, Wavelength=280 nm

| Retention time [min] | Type | Peak width [min] | Peak area | Peak height | Peak area% |
|---|---|---|---|---|---|
| 23.554 | HH | 9.21 | 3821.14 | 53.33 | 99.34 |
| 32.164 | HH | 4.26 | 25.53 | 0.19 | 0.66 |
| | | **Total** | 3846.67 | | |

FIG. 2

FIG. 3

FIG. 4

A

B

FIG. 5

FIG. 6

FIG. 7

Anti-PEG IgG S/N value

FIG. 8

Anti-PEG IgM S/N value

FIG. 9

FIG. 10

FIG. 11

FIG. 12

## INTERNATIONAL SEARCH REPORT

| | International application No. |
|---|---|
| | **PCT/CN2024/091015** |

| **A.** | **CLASSIFICATION OF SUBJECT MATTER** |
|---|---|

C12N9/88(2006.01)i; C12N9/96(2006.01)i; A61K38/43(2006.01)i

According to International Patent Classification (IPC) or to both national classification and IPC

| **B.** | **FIELDS SEARCHED** |
|---|---|

Minimum documentation searched (classification system followed by classification symbols)

IPC:C12N,A61K

Documentation searched other than minimum documentation to the extent that such documents are included in the fields searched

Electronic data base consulted during the international search (name of data base and, where practicable, search terms used)

CNTXT, ENTXTC, VCN, VEN, PUBMED, NCBI, CNKI, BAIDU, BING, STN: 苯丙氨酸裂解酶, PAL, phenylalanine ammnia lyase, 突变, 变体, mutant, 72, 240, 467, 544, 聚乙二醇, PEG, 序列1-11, sequences 1-11

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| A | CN 101842482 A (BIOMARIN PHARMACEUTICIAL INC. et al.) 22 September 2010 (2010-09-22)<br>entire document | 1-62 |
| A | CN 102753566 A (BIOMARIN PHARMACEUTICIAL INC.) 24 October 2012 (2012-10-24)<br>entire document | 1-62 |
| A | CN 106834261 A (BIOMARIN PHARMACEUTICIAL INC.) 13 June 2017 (2017-06-13)<br>entire document | 1-62 |
| A | CN 115873837 A (ANHUI YINGKE BIOTECHNOLOGY CO., LTD.) 31 March 2023 (2023-03-31)<br>entire document | 1-62 |
| A | CN 106636048 A (JIANGNAN UNIVERSITY) 10 May 2017 (2017-05-10)<br>entire document | 1-62 |
| A | US 2009047265 A1 (BIOMARIN PHARMACEUTICAL INC.) 19 February 2009 (2009-02-19)<br>entire document | 1-62 |

☑ Further documents are listed in the continuation of Box C.     ☑ See patent family annex.

| | |
|---|---|
| *   Special categories of cited documents:<br>"A"   document defining the general state of the art which is not considered to be of particular relevance<br>"D"   document cited by the applicant in the international application<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **19 July 2024** | **25 July 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
|---|
| **PCT/CN2024/091015** |

### C. DOCUMENTS CONSIDERED TO BE RELEVANT

| Category* | Citation of document, with indication, where appropriate, of the relevant passages | Relevant to claim No. |
|---|---|---|
| A | US 2007048855 A1 (GAMEZ, A. et al.) 01 March 2007 (2007-03-01)<br>entire document | 1-62 |
| A | LEVY, H.L. et al. "Phenylalanine Ammonia Lyase (PAL): From Discovery to Enzyme Substitution Therapy for Phenylketonuria"<br>*Molecular Genetics and Metabolism*, 09 June 2018 (2018-06-09),<br>pp. 223-229 | 1-62 |

Form PCT/ISA/210 (second sheet) (July 2022)

## INTERNATIONAL SEARCH REPORT

| International application No. |
|---|
| **PCT/CN2024/091015** |

**Box No. I     Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed.

    b.  ☐ furnished subsequent to the international filing date for the purposes of international search (Rule 13*ter*.1(a)),

        ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

## INTERNATIONAL SEARCH REPORT

| International application No. |
| --- |
| **PCT/CN2024/091015** |

**Box No. II      Observations where certain claims were found unsearchable (Continuation of item 2 of first sheet)**

This international search report has not been established in respect of certain claims under Article 17(2)(a) for the following reasons:

1. ☑  Claims Nos.: **47, 49**
   because they relate to subject matter not required to be searched by this Authority, namely:

   Claims 47 and 49 respectively relate to the use of a product such as the mutant in the prevention and/ or treatment of homophenylalanine-related diseases, and a method for preventing and/or treating homophenylalanine-related diseases, which fall within methods for treatment of diseases as defined in PCT Rule 39.1(iv). The present report is provided on the basis of a corresponding pharmaceutical use.

2. ☐  Claims Nos.:
   because they relate to parts of the international application that do not comply with the prescribed requirements to such an extent that no meaningful international search can be carried out, specifically:

3. ☐  Claims Nos.:
   because they are dependent claims and are not drafted in accordance with the second and third sentences of Rule 6.4(a).

Form PCT/ISA/210 (continuation of first sheet) (July 2022)

| INTERNATIONAL SEARCH REPORT | | | International application No. | | | |
|---|---|---|---|---|---|---|
| Information on patent family members | | | PCT/CN2024/091015 | | | |

| Patent document cited in search report | | | Publication date (day/month/year) | Patent family member(s) | | | Publication date (day/month/year) |
|---|---|---|---|---|---|---|---|
| CN | 101842482 | A | 22 September 2010 | AU | 2008263190 | A1 | 18 December 2008 |
| | | | | AU | 2008263190 | B2 | 17 April 2014 |
| | | | | KR | 20150038753 | A | 08 April 2015 |
| | | | | IL | 202132 | A0 | 16 June 2010 |
| | | | | IL | 202132 | A | 29 June 2017 |
| | | | | BRPI | 0811267 | A2 | 07 October 2014 |
| | | | | BRPI | 0811267 | B1 | 10 November 2020 |
| | | | | BRPI | 0811267 | B8 | 25 May 2021 |
| | | | | HRP | 20151268 | T1 | 18 December 2015 |
| | | | | DK | 2152868 | T3 | 16 November 2015 |
| | | | | PL | 2152868 | T3 | 29 January 2016 |
| | | | | MX | 2009012453 | A | 25 January 2010 |
| | | | | KR | 20100024437 | A | 05 March 2010 |
| | | | | KR | 101603796 | B1 | 17 March 2016 |
| | | | | EA | 200970980 | A1 | 30 April 2010 |
| | | | | EA | 018443 | B1 | 30 August 2013 |
| | | | | NO | 2019037 | I1 | 16 October 2019 |
| | | | | LUC | 00133 | I1 | 16 October 2019 |
| | | | | LUC | 00133 | I2 | 16 July 2020 |
| | | | | CY | 2019039 | I1 | 29 May 2020 |
| | | | | PE | 20090315 | A1 | 19 March 2009 |
| | | | | PT | 2152868 | E | 13 November 2015 |
| | | | | TW | 200902052 | A | 16 January 2009 |
| | | | | TWI | 418633 | B | 11 December 2013 |
| | | | | AR | 066716 | A1 | 09 September 2009 |
| | | | | HUS | 1900049 | I1 | 28 November 2019 |
| | | | | WO | 2008153776 | A1 | 18 December 2008 |
| | | | | WO | 2008153776 | A8 | 30 July 2009 |
| | | | | CA | 2687450 | A1 | 18 December 2008 |
| | | | | CA | 2687450 | C | 26 March 2019 |
| | | | | HUE | 025784 | T2 | 30 May 2016 |
| | | | | AR | 110834 | A2 | 08 May 2019 |
| | | | | JP | 2010529835 | A | 02 September 2010 |
| | | | | JP | 5670183 | B2 | 18 February 2015 |
| | | | | EP | 2657335 | A1 | 30 October 2013 |
| | | | | CY | 1116894 | T1 | 05 April 2017 |
| | | | | CL | 2008001497 | A1 | 09 January 2009 |
| | | | | LTPA | 2019517 | I1 | 25 October 2019 |
| | | | | LTC | 2152868 | I2 | 25 September 2020 |
| | | | | US | 2008008695 | A1 | 10 January 2008 |
| | | | | US | 7534595 | B2 | 19 May 2009 |
| | | | | ES | 2551315 | T3 | 18 November 2015 |
| | | | | NL | 301011 | I1 | 16 October 2019 |
| | | | | NL | 301011 | I2 | 13 May 2020 |
| | | | | EP | 2152868 | A1 | 17 February 2010 |
| | | | | EP | 2152868 | B1 | 02 September 2015 |
| | | | | HK | 1135141 | A1 | 28 May 2010 |
| | | | | SI | 2152868 | T1 | 31 December 2015 |
| | | | | MY | 151413 | A | 30 May 2014 |
| CN | 102753566 | A | 24 October 2012 | ES | 2690542 | T3 | 21 November 2018 |

Form PCT/ISA/210 (patent family annex) (July 2022)

## INTERNATIONAL SEARCH REPORT
### Information on patent family members

International application No.

**PCT/CN2024/091015**

| Patent document cited in search report | | | Publication date (day/month/year) | Patent family member(s) | | | Publication date (day/month/year) |
|---|---|---|---|---|---|---|---|
| | | | | CY | 1120768 | T1 | 11 December 2019 |
| | | | | EP | 2531209 | A2 | 12 December 2012 |
| | | | | EP | 2531209 | A4 | 03 July 2013 |
| | | | | EP | 2531209 | B1 | 20 April 2016 |
| | | | | ES | 2582459 | T3 | 13 September 2016 |
| | | | | DK | 3025728 | T3 | 29 October 2018 |
| | | | | PL | 3025728 | T3 | 31 December 2018 |
| | | | | JP | 2013518893 | A | 23 May 2013 |
| | | | | JP | 5828844 | B2 | 09 December 2015 |
| | | | | CA | 2782444 | A1 | 11 August 2011 |
| | | | | CA | 2782444 | C | 23 October 2018 |
| | | | | PT | 3025728 | T | 05 November 2018 |
| | | | | SI | 3025728 | T1 | 30 November 2018 |
| | | | | IL | 221099 | B | 28 February 2018 |
| | | | | US | 2016362675 | A1 | 15 December 2016 |
| | | | | US | 10221408 | B2 | 05 March 2019 |
| | | | | IL | 257437 | A | 30 April 2018 |
| | | | | IL | 257437 | B | 30 June 2019 |
| | | | | US | 2013039898 | A1 | 14 February 2013 |
| | | | | EP | 3479842 | A2 | 08 May 2019 |
| | | | | EP | 3479842 | A3 | 17 July 2019 |
| | | | | EP | 4062928 | A2 | 28 September 2022 |
| | | | | EP | 4062928 | A3 | 30 November 2022 |
| | | | | AU | 2011212929 | A1 | 21 June 2012 |
| | | | | AU | 2011212929 | B2 | 27 October 2016 |
| | | | | LT | 3025728 | T | 25 October 2018 |
| | | | | US | 2019345475 | A1 | 14 November 2019 |
| | | | | US | 11505790 | B2 | 22 November 2022 |
| | | | | US | 2024035013 | A1 | 01 February 2024 |
| | | | | HUE | 039516 | T2 | 28 January 2019 |
| | | | | HRP | 20181602 | T1 | 30 November 2018 |
| | | | | HK | 1225299 | B | 08 September 2017 |
| | | | | WO | 2011097335 | A2 | 11 August 2011 |
| | | | | WO | 2011097335 | A3 | 20 October 2011 |
| | | | | EP | 3025728 | A1 | 01 June 2016 |
| | | | | EP | 3025728 | B1 | 08 August 2018 |
| CN | 106834261 | A | 13 June 2017 | None | | | |
| CN | 115873837 | A | 31 March 2023 | None | | | |
| CN | 106636048 | A | 10 May 2017 | None | | | |
| US | 2009047265 | A1 | 19 February 2009 | PT | 2175875 | T | 21 November 2016 |
| | | | | US | 2009263369 | A1 | 22 October 2009 |
| | | | | US | 7790433 | B2 | 07 September 2010 |
| | | | | LT | 2175875 | T | 12 December 2016 |
| | | | | TW | 200911283 | A | 16 March 2009 |
| | | | | TWI | 418787 | B | 11 December 2013 |
| | | | | HRP | 20161474 | T1 | 30 December 2016 |
| | | | | SI | 2175875 | T1 | 31 January 2017 |
| | | | | AR | 117389 | A2 | 04 August 2021 |
| | | | | HUE | 029510 | T2 | 28 February 2017 |
| | | | | HUE | 029510 | T4 | 29 May 2017 |

Form PCT/ISA/210 (patent family annex) (July 2022)

**INTERNATIONAL SEARCH REPORT**
Information on patent family members

| | | | International application No. |
|---|---|---|---|
| | | | **PCT/CN2024/091015** |

| Patent document cited in search report | | | Publication date (day/month/year) | Patent family member(s) | | | Publication date (day/month/year) |
|---|---|---|---|---|---|---|---|
| | | | | RU | 2012134838 | A | 20 March 2014 |
| | | | | RU | 2553343 | C2 | 10 June 2015 |
| | | | | ES | 2602618 | T3 | 21 February 2017 |
| | | | | PE | 20090605 | A1 | 16 May 2009 |
| | | | | US | 7537923 | B2 | 26 May 2009 |
| | | | | CY | 1118222 | T1 | 28 June 2017 |
| | | | | EP | 2175875 | A2 | 21 April 2010 |
| | | | | EP | 2175875 | B1 | 05 October 2016 |
| | | | | JP | 2010536759 | A | 02 December 2010 |
| | | | | JP | 5584120 | B2 | 03 September 2014 |
| | | | | US | 2010278802 | A1 | 04 November 2010 |
| | | | | DK | 2175875 | T3 | 21 November 2016 |
| | | | | PL | 2175875 | T3 | 28 April 2017 |
| | | | | CA | 2687028 | A1 | 26 February 2009 |
| | | | | CA | 2687028 | C | 16 February 2016 |
| | | | | WO | 2009025760 | A2 | 26 February 2009 |
| | | | | WO | 2009025760 | A3 | 25 June 2009 |
| | | | | AR | 067972 | A1 | 28 October 2009 |
| | | | | US | 2009047268 | A1 | 19 February 2009 |
| | | | | US | 7560263 | B2 | 14 July 2009 |
| | | | | BRPI | 0811589 | A2 | 29 October 2014 |
| | | | | BRPI | 0811589 | B1 | 12 May 2020 |
| | | | | BRPI | 0811589 | B8 | 25 May 2021 |
| | | | | BR | 122018072594 | B1 | 27 October 2020 |
| | | | | BR | 122018072594 | B8 | 27 July 2021 |
| | | | | MX | 2009012261 | A | 15 March 2010 |
| | | | | RU | 2009141987 | A | 20 May 2011 |
| | | | | CL | 2008002390 | A1 | 29 May 2009 |
| | | | | AU | 2008289549 | A1 | 26 February 2009 |
| | | | | AU | 2008289549 | B2 | 06 February 2014 |
| US | 2007048855 | A1 | 01 March 2007 | EP | 1814984 | A2 | 08 August 2007 |
| | | | | CA | 2580679 | A1 | 30 March 2006 |
| | | | | AU | 2005286763 | A1 | 30 March 2006 |
| | | | | WO | 2006034373 | A2 | 30 March 2006 |
| | | | | WO | 2006034373 | A9 | 11 May 2006 |
| | | | | WO | 2006034373 | A8 | 10 August 2006 |
| | | | | WO | 2006034373 | A3 | 02 November 2006 |
| | | | | JP | 2008513027 | A | 01 May 2008 |
| | | | | US | 7553653 | B2 | 30 June 2009 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Current Protocols in Molecular Biology. Greene Publishing and Wiley-Interscience, 1995
- **DAYHOFF**. Atlas of Protein Sequence and Structure. National Biomedical Research Foundation, 1978, vol. 5
- **NEEDLEMAN et al.** *J. Mol. Biol.*, 1970, vol. 48, 443
- **SMITH et al.** *Adv. Appl. Math.*, 1981, vol. 2, 482
- **PEARSON et al.** *Proc. Natl. Acad. Sci.*, 1988, vol. 85, 2444

- **SMITH** ; **WATERMAN**. *Meth. Mol. Biol.*, 1997, vol. 70, 173-187
- **ALTSCHUL et al.** *J. Mol. Biol.*, 1990, vol. 215, 403-410
- **ALTSCHUL et al.** *Meth. Enzym.*, 1996, vol. 266, 460-480
- **SAMBROOK et al.** Molecular Cloning: A Laboratory Manual. 1989
- Oligonucleotide Synthesis. 1984
- **JUDY PEAT et al.** *Determination of Phenylalanine and Tyrosine by High Performance Liquid Chromatography-Tandem Mass Spectrometry*